# EUROPEAN PATENT APPLICATION

(11) **EP 4 371 994 A1**
(43) Date of publication of application: **22.05.2024**
(21) Application number: 22837554.9
(22) Date of filing: 29.06.2022
(51) Int. Cl.: C07F 15/00, C09K 11/06, H10K 59/00, H10K 50/00, H05B 33/02, C07D 405/04

(54) **ORGANIC METAL COMPLEX, AND ORGANIC LIGHT-EMITTING ELEMENT, DISPLAY DEVICE, IMAGING DEVICE, ELECTRONIC EQUIPMENT, LIGHTING DEVICE AND MOBILE OBJECT EACH CONTAINING SAME**

(30) Priority: 06.07.2021 JP 2021112295
(71) Applicant: CANON KABUSHIKI KAISHA, Tokyo 146-8501 (JP)
(72) Inventor: TANI Yutaka, Tokyo 146-8501 (JP); HOTTA Katsuyuki, Tokyo 146-8501 (JP); HANDA Satoshi, Tokyo 146-8501 (JP)
(74) Representative: TBK
(86) International application number: PCT/JP2022/025913
(87) International publication number: WO 2023/282138

(57) **Abstract**

The present disclosure provides an organometallic complex represented by the following general formula (1).

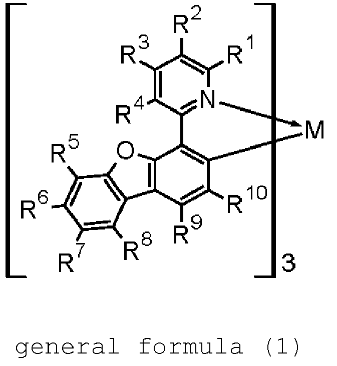

In general formula (1), M is a metal atom selected from Ir, Pt, Os, Rh, Pd, and Ru. R¹ to R⁸ are each independently selected from a hydrogen atom and a substituent. At least one of R¹ to R⁸ is the substituent, or at least any of R⁹ and R¹⁰ is the alkyl group having two or less carbon atoms.

## Description

### Technical Field

The present invention relates to an organometallic complex, and an organic light-emitting device, a display apparatus, an image pickup apparatus, an electronic apparatus, a lighting apparatus, and a moving object containing the organometallic complex.

### Background Art

Organic light-emitting devices are electronic devices each including a first electrode, a second electrode, and an organic compound layer disposed between these electrodes. The injection of electrons and holes from these pairs of electrodes into the organic compound layer generates excitons in the light-emitting organic compound in the organic compound layer, and when the excitons return to the ground state, the organic light-emitting device emits light. Organic light-emitting devices are also referred to as organic electroluminescent devices or organic EL devices.

Light-emitting organic compounds can be broadly classified into two types based on their luminescence principle: fluorescent materials and phosphorescent materials. In electrical exciton generation within organic light-emitting devices, phosphorescent materials are known to exhibit higher luminous efficiencies than fluorescent materials because of the principles of quantum mechanics. Specifically, Ir(ppy)₃, which is an organometallic complex represented by the following structure, is known as a green phosphorescent material.

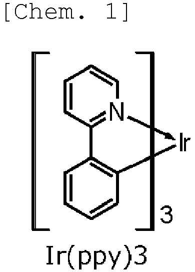

New materials have been developed for organic light-emitting devices to improve, for example, the driving voltage, emission quantum yield, color gamut, and device life. Patent Literature 1 describes the following compound A as a compound for a light-emitting device that maintains high luminance for a long period of time and has little deterioration due to electrification. Patent Literature 2 describes an organometallic complex having a ligand with the same structure as compound A as a compound that phosphoresces. Patent Document 3 describes a material having an alkyl group or a cycloalkyl group having 3 to 10 carbon atoms at the 1-position of dibenzofuran in a ligand in order to improve the lifetime or efficiency of an organic light-emitting device. As specific examples, Compounds B and C are described below. Patent Literature 4 describes compound D as a complex for improving efficiency, operating voltage, lifetime, and color coordinates.

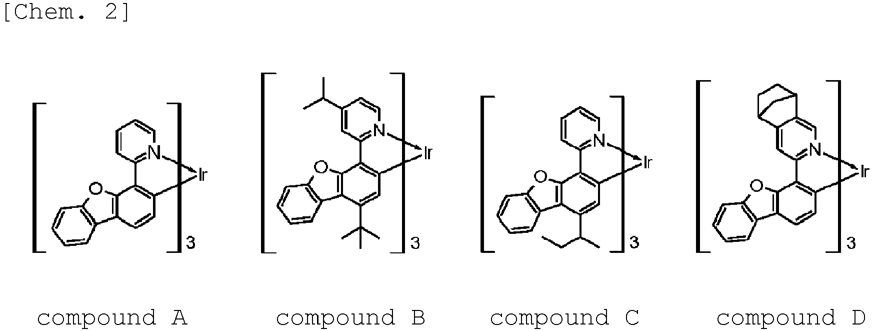

### Citation List

### Patent Literature

PTL 1: Japanese Patent Laid-Open No. 2002-332291
PTL 2: U.S. Patent Application Publication No. 2018/0282356
PTL 3: International Publication No. 2019/221484
PTL 4: International Publication No. 2014/023377

### Summary of Invention

### Technical Problem

In organic light-emitting devices, a phenomenon called roll-off in which the luminous efficiency is reduced at a high current density is known. The organometallic complexes described in Patent Literatures 1 to 4 have room for improvement with respect to a decrease in luminous efficiency at a second current density higher than a first current density based on luminous efficiency at the first current density, that is, what is called roll-off characteristics.

### Solution to Problem

The present invention has been accomplished in view of the above problems, and it is an object of the present invention to provide an organometallic complex in which the rate of decrease in luminous efficiency at a second current density higher than a first current density is reduced with respect to the luminous efficiency at the first current density.

The present invention provides an organometallic complex represented by the following general formula (1).

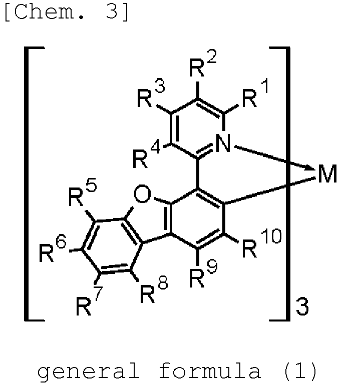

In general formula (1), M is a metal atom selected from Ir, Pt, Os, Rh, Pd, and Ru. R¹ to R⁸ are each independently selected from a hydrogen atom and a substituent. The substituent is a halogen atom, a cyano group, a nitro group, a trialkylsilyl group, a substituted or unsubstituted alkyl group, a substituted or unsubstituted cycloalkyl group, or a substituted or unsubstituted aryl group having two or less rings. R⁹ and R¹⁰ are each independently selected from a hydrogen atom and an alkyl group having two or less carbon atoms. However, at least one of R¹ to R⁸ is the substituent, or at least any of R⁹ and R¹⁰ is the alkyl group having two or less carbon atoms.

### Advantageous Effects of Invention

According to the present invention, it is possible to provide an organometallic complex in which the rate of decrease in luminous efficiency at a second current density higher than a first current density is reduced with respect to the luminous efficiency at the first current density. Brief Description of Drawings

[Fig. 1A] Fig. 1A is a schematic cross-sectional view of an example of a pixel of a display apparatus according to an embodiment of the present invention.
[Fig. 1B] Fig. 1B is a schematic cross-sectional view of an example of a display apparatus including organic light-emitting devices according to an embodiment of the present invention.
[Fig. 2] Fig. 2 is a schematic view of an example of a display apparatus including organic light-emitting devices according to an embodiment of the present invention.
[Fig. 3A] Fig. 3A is a schematic view of an example of an image pickup apparatus according to an embodiment of the present invention.
[Fig. 3B] Fig. 3B is a schematic view of an example of a portable apparatus according to an embodiment of the present invention.
[Fig. 4A] Fig. 4A is a schematic view of an example of a display apparatus according to an embodiment of the present invention.
[Fig. 4B] Fig. 4B is a schematic view of an example of a foldable display apparatus.
[Fig. 5A] Fig. 5A is a schematic view of an example of a lighting apparatus according to an embodiment of the present invention.
[Fig. 5B] Fig. 5B is a schematic view of an automobile as an example of a moving object according to an embodiment of the present invention.
[Fig. 6A] Fig. 6A is a schematic view illustrating an example of a wearable device according to an embodiment of the present invention.
[Fig. 6B] Fig. 6B is a schematic view of an example of a wearable device according to an embodiment of the present invention, the wearable device including an image pickup apparatus.

### Description of Embodiments

Embodiments of the present invention will be described below. The present invention is not limited by the description below, and one skilled in the art could easily recognize that various changes in form and detail can be made without departing from the spirit and the scope of the present invention. That is, the present invention should not be construed as being limited to the following description.

### [Organometallic Complex Represented by General Formula (1)]

The inventors have conducted studies and have found an organometallic complex in which a decrease in light-emitter efficiency is reduced even at a high current density and which is represented by the following general formula (1).

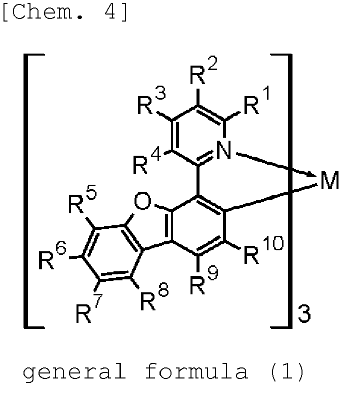

In general formula (1), M is a metal atom selected from Ir, Pt, Os, Rh, Pd, and Ru. R¹ to R⁸ are each independently selected from a hydrogen atom and a substituent. The substituent is a halogen atom, a cyano group, a nitro group, a trialkylsilyl group, a substituted or unsubstituted alkyl group, a substituted or unsubstituted cycloalkyl group, and a substituted or unsubstituted aryl group having two or less rings.

R⁹ and R¹⁰ are each independently selected from a hydrogen atom and an alkyl group having two or less carbon atoms.

However, at least one of R¹ to R⁸ is the substituent, or at least any of R⁹ and R¹⁰ is the alkyl group having two or less carbon atoms.

In the present specification, M is a metal atom that forms a complex, and specific examples thereof include Ir, Pt, Os, Rh, Pd, Ru, and Re. Among these, Ir is preferred.

In this specification, the halogen atom of R¹ to R⁸ is any of a fluorine atom, a chlorine atom, a bromine atom, and an iodine atom. Among these, a fluorine atom is preferred from the viewpoint of thermal stability.

In this specification, alkyl groups in trialkylsilyl groups as R¹ to R⁸ may be alkyl groups each having an independent number of carbon atoms. The alkyl group is preferably an alkyl group having 1 to 8 carbon atoms, and may be a linear alkyl group or a branched alkyl group. More specific examples thereof include, but are not limited to, a trimethylsilyl group, a tert-butyldimethylsilyl group, and a triisopropylsilyl group.

In this specification, each of the alkyl groups of R¹ to R⁸ may be a linear alkyl group or a branched alkyl group. The alkyl group may have 1 to 20 carbon atoms, and may have 1 to 8 carbon atoms.

In this specification, each of the cycloalkyl groups of R¹ to R⁸ and R¹¹ to R¹⁸ may have 3 to 20 carbon atoms, and may have 3 to 10 carbon atoms. The cycloalkyl group is preferably cyclohexane or cyclopentane. A carbon atom of the cycloalkyl group may be replaced with an oxygen atom, but it is not preferable to replace two consecutive carbon atoms with oxygen atoms. One carbon atom may be replaced with an oxygen atom.

In this specification, examples of the aryl group having two or less rings include a phenyl group, a naphthyl group, a pyridyl group, a benzothienyl group, a benzofuryl group, a benzoxazolyl group, a quinolinyl group, and an isoquinolinyl group. A phenyl group is preferred.

In this specification, examples of the aryl group include a phenyl group, a naphthyl group, a biphenyl group, a fluorenyl group, a phenanthrenyl group, an anthracenyl group, a pyridine group, a carbazolyl group, a dibenzofuryl group, and a dibenzothienyl group.

In this specification, the alkyl group or the cycloalkyl group may further have a substituent. Examples of the substituent include a halogen atom, a cyano group, and a nitro group. The halogen atom with which the alkyl group can be substituted is any of a fluorine atom, a chlorine atom, a bromine atom, and an iodine atom. Among these, a fluorine atom is preferred from the viewpoint of thermal stability. In the alkyl group or cycloalkyl group, one methylene group or two or more methylene groups that are not adjacent to each other can be substituted with an -O-group, a -S- group, a -C(=O)- group, a -C(=O)O- group, an - O(C=O)- group, a -CH=CH- group, or a -C=C- group, and a hydrogen atom can be replaced with a fluorine atom.

In this specification, the aryl group may have an alkyl group as a substituent. In the alkyl group, one methylene group or two or more methylene groups that are not adjacent to each other can be substituted with an -O- group, a -S-group, a -C(=O)- group, a -C(=O)O- group, an -O(C=O)- group, a -CH=CH - group, or a -C=C- group, and a hydrogen atom can be replaced with a fluorine atom.

Specific examples of the aryl group in this specification, including those further having a substituent, include, but are not limited to, a phenyl group, a 1-naphthyl group, a 2-naphthyl group, a 4-biphenyl group, a 2-fluorenyl group, a 9-phenanthryl group, a 2-anthryl group, a 1-pyrenyl group, a 1-imidazolyl group, a 2-furyl group, a 3-benzofuryl group, a 4-dibenzofuryl group, a 2-thienyl group, a 3-benzothienyl group, a 2-dibenzothienyl group, a 2-pyridyl group, a 2-pyrimidinyl group, a 1-indolyl group, a 2-indolyl group, a 9-carbazolyl group, a p-chlorophenyl group, an o-tolyl group, a 4-methoxyphenyl group, a 4'-(1-hexynyl) phenyl group, a 2-(1-(1,1,2,2,3,3,4,4,5,5,6,6,7,7,8,8-hexadecafluoro)octyloxycarbonyl)phenyl group, a 4'-cyanobiphenyl group, a 2-(9,9-dimethyl)fluorenyl group, and a 3-(9,9-dioctyl)fluorenyl group.

R⁹ and R¹⁰ are each independently selected from a hydrogen atom and an alkyl group having two or less carbon atoms. Specifically, each of them is a hydrogen atom, a methyl group, or an ethyl group.

In the organometallic complex according to the present invention, at least one of R¹ to R⁸ is any of the substituents described above, or at least any of R⁹ and R¹⁰ is an alkyl group having two or less carbon atoms. Thus, the organometallic complex according to the present invention has the effect of reducing a decrease in luminous efficiency at a high current density. That is, the organometallic complex has excellent roll-off characteristics.

When at least one of R¹ to R⁸ is a substituent, both of R⁹ and R¹⁰ may be hydrogen atoms.

R¹ to R⁴ may be each independently selected from a hydrogen atom and the alkyl group described above. Preferably, R³ is a tert-butyl group.

R⁵ to R⁸ may be each independently selected from a hydrogen atom and the alkyl group described above.

In the case of R³ in general formula (1), the aryl group can be used without being limited to two or less rings. That is, general formula (1) may be in the following range.

R¹, R², and R⁴ to R⁸ are each independently selected from a hydrogen atom and a substituent. The substituent is a halogen atom, a cyano group, a nitro group, a trialkylsilyl group, a substituted or unsubstituted alkyl group, a substituted or unsubstituted cycloalkyl group, or a substituted or unsubstituted phenyl group. R³ is selected from a hydrogen atom, a halogen atom, a cyano group, a nitro group, a trialkylsilyl group, a substituted or unsubstituted alkyl group, a substituted or unsubstituted cycloalkyl group, and a substituted or unsubstituted aryl group. R⁹ and R¹⁰ are each independently selected from a hydrogen atom and an alkyl group having two or less carbon atoms. However, R¹ to R⁸ are not hydrogen atoms, or at least any of R⁹ and R¹⁰ is the alkyl group having two or less carbon atoms.

When at least any of R⁹ and R¹⁰ in general formula (1) is an alkyl group having two or less carbon atoms, an aryl group can be used for R¹ to R⁸ without being limited to two or less rings. When any of R⁹ and R¹⁰ is an alkyl group having two or less carbon atoms, R⁹ is preferably the alkyl group having two or less carbon atoms. That is, general formula (1) may be in the following range.

R¹ to R⁸ are each independently selected from a hydrogen atom and a substituent. The substituent is a halogen atom, a cyano group, a nitro group, a trialkylsilyl group, a substituted or unsubstituted alkyl group, a substituted or unsubstituted cycloalkyl group, and a substituted or unsubstituted aryl group. R⁹ and R¹⁰ are each independently selected from a hydrogen atom and an alkyl group having two or less carbon atoms. However, at least any of R⁹ and R¹⁰ is the alkyl group having two or less carbon atoms.

Specific examples of the structural formula of the organometallic complex of the present invention are illustrated below. An iridium complex is given as an example, but the same applies when another metal is used. The number of ligands may vary in accordance with the coordination number of the metal.

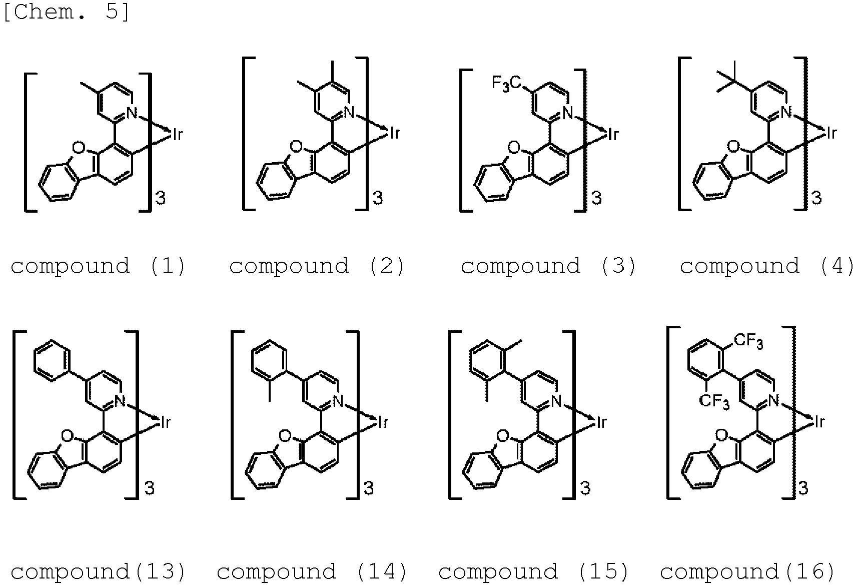

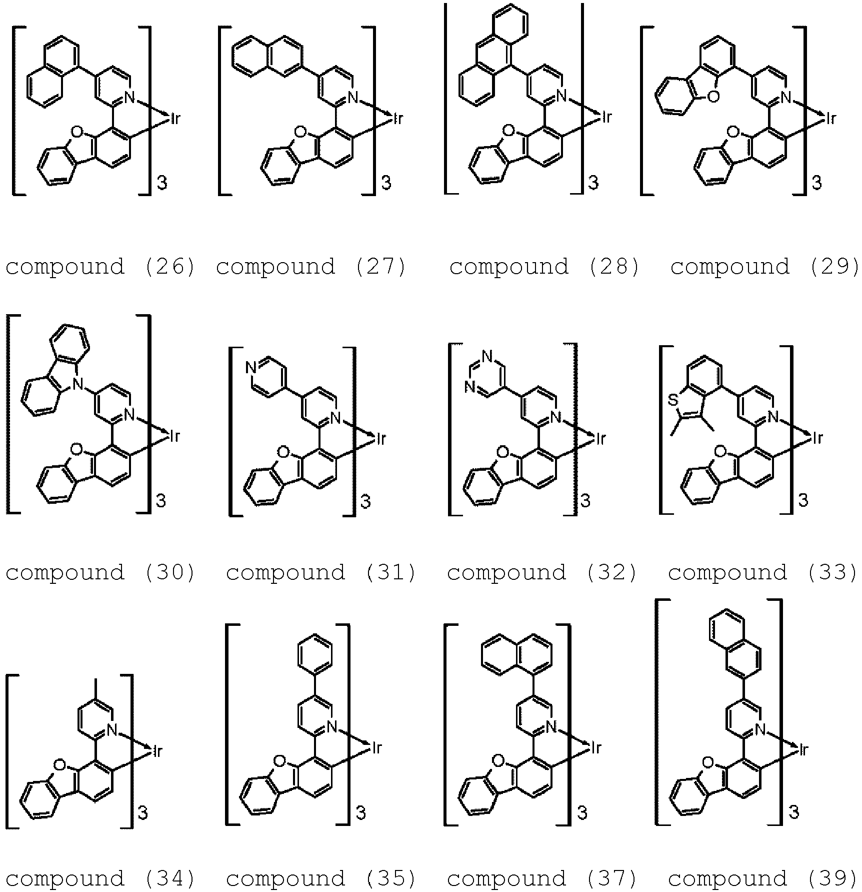

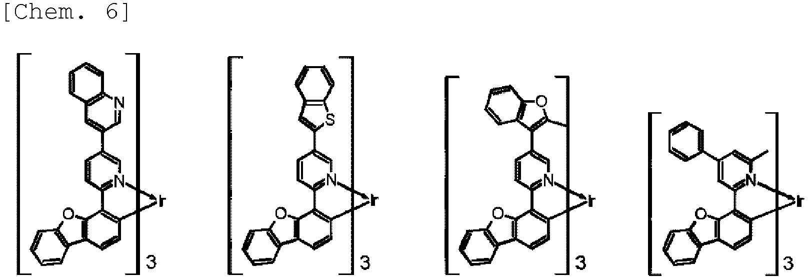

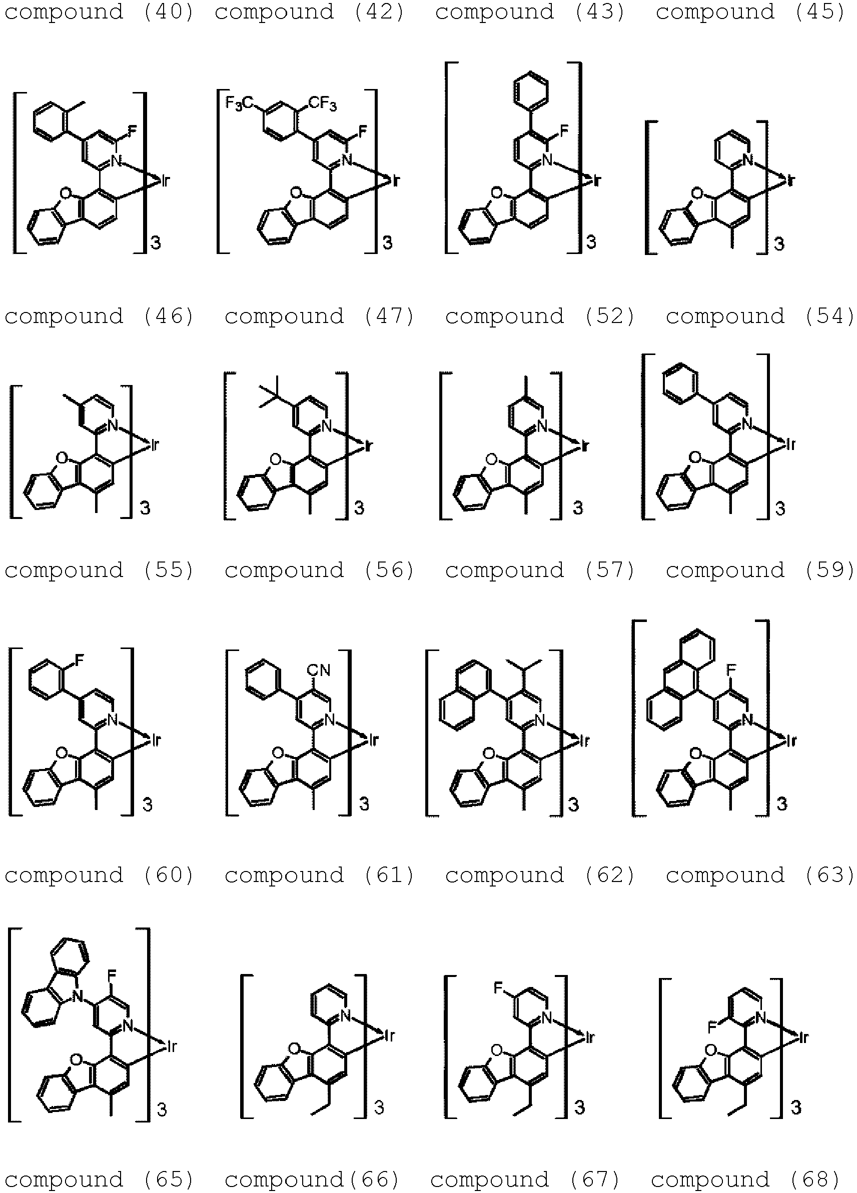

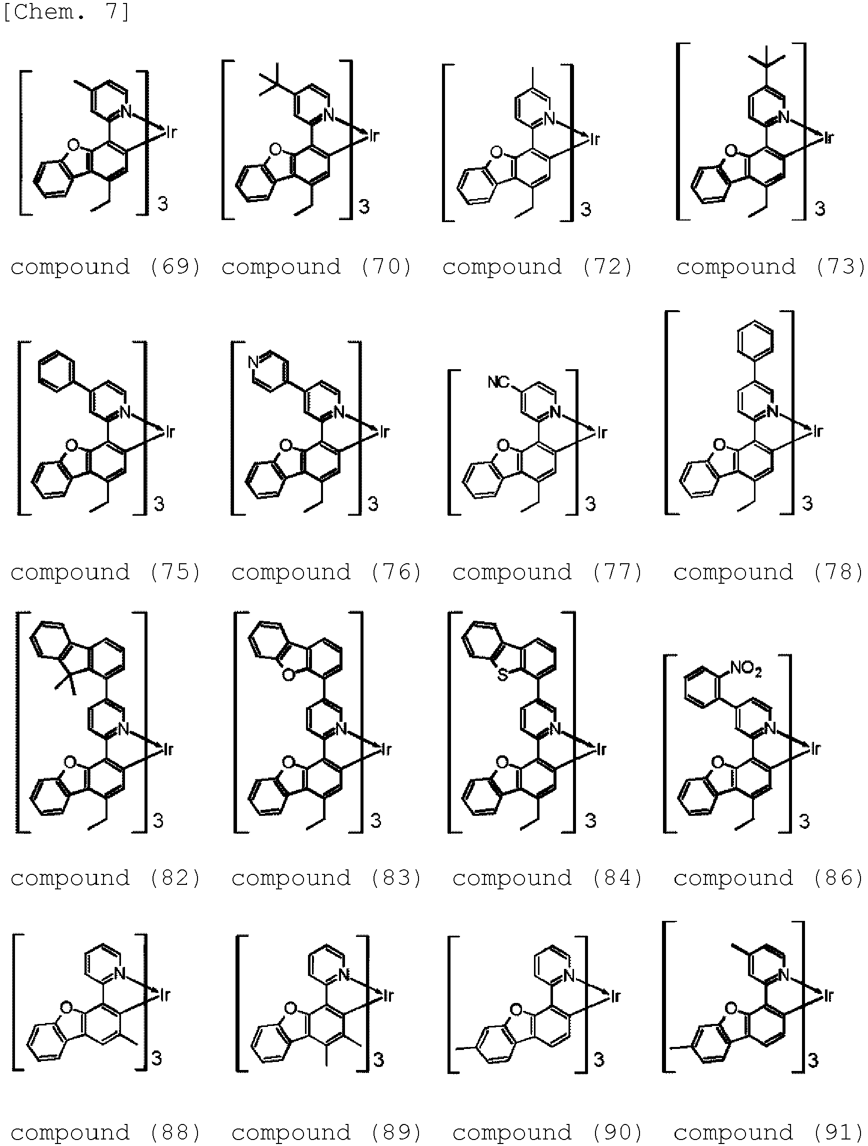

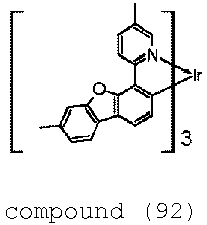

Among the exemplified compounds, the following exemplified compounds have high solvent solubility and thus can be preferably used in a film formation method by an application method.

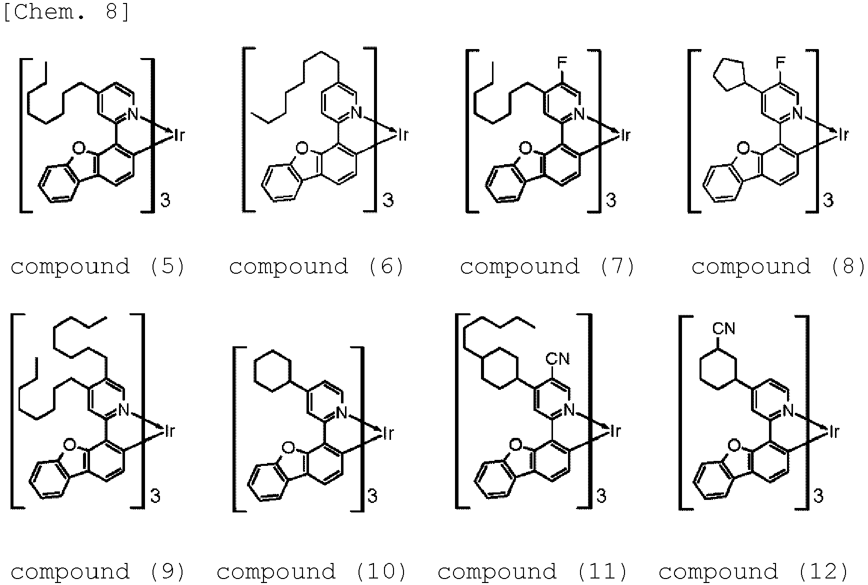

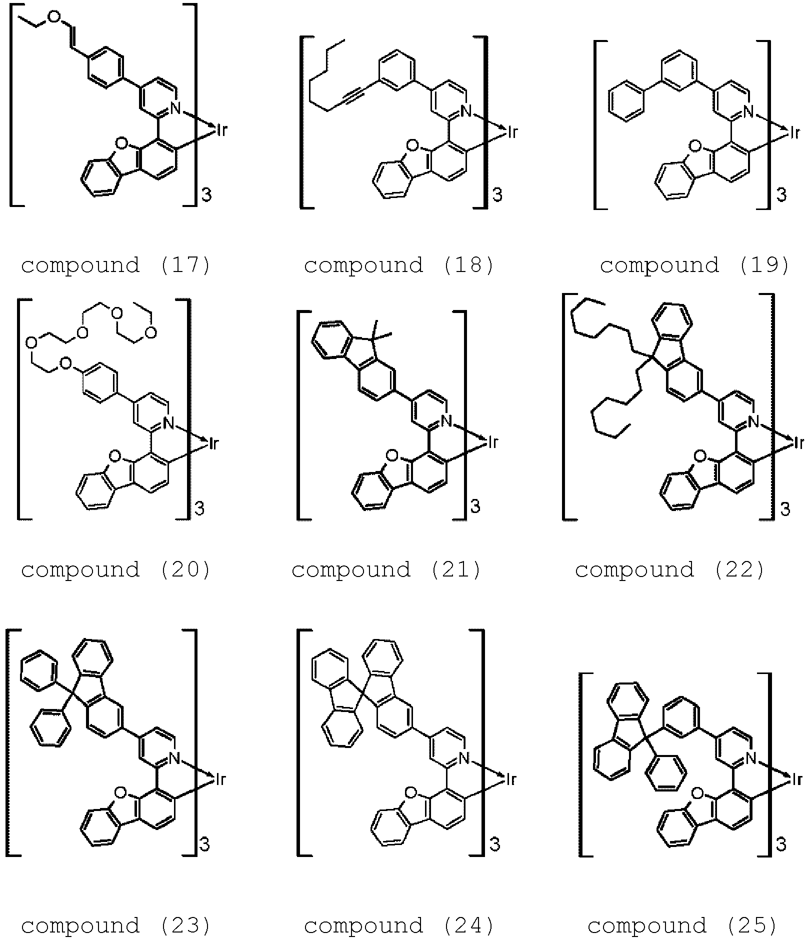

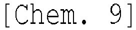

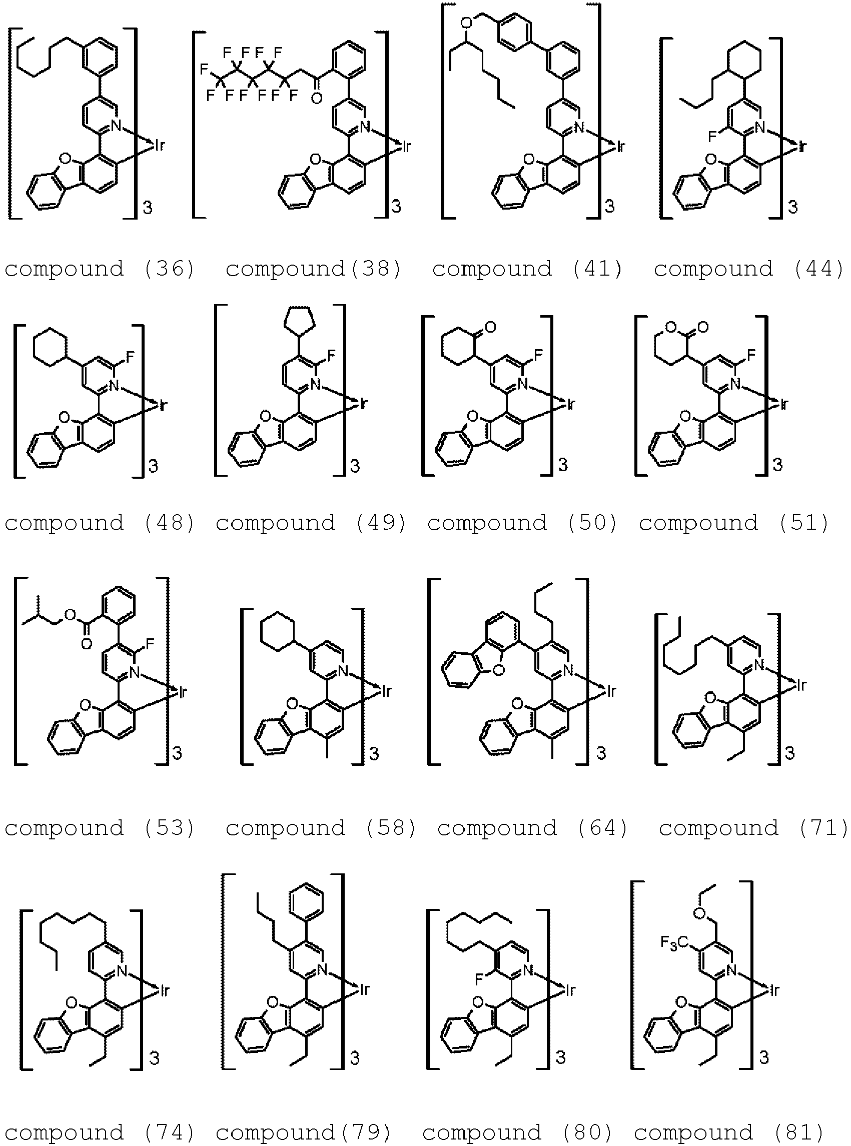

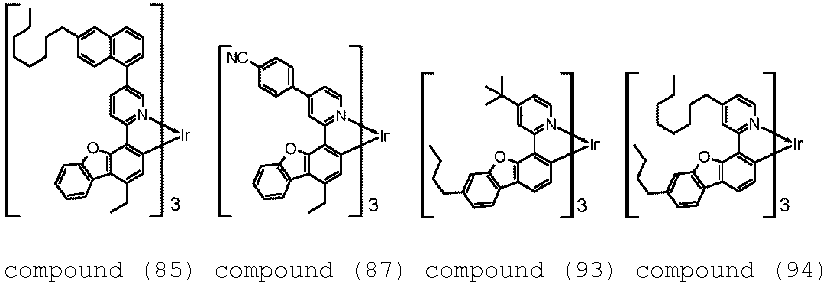

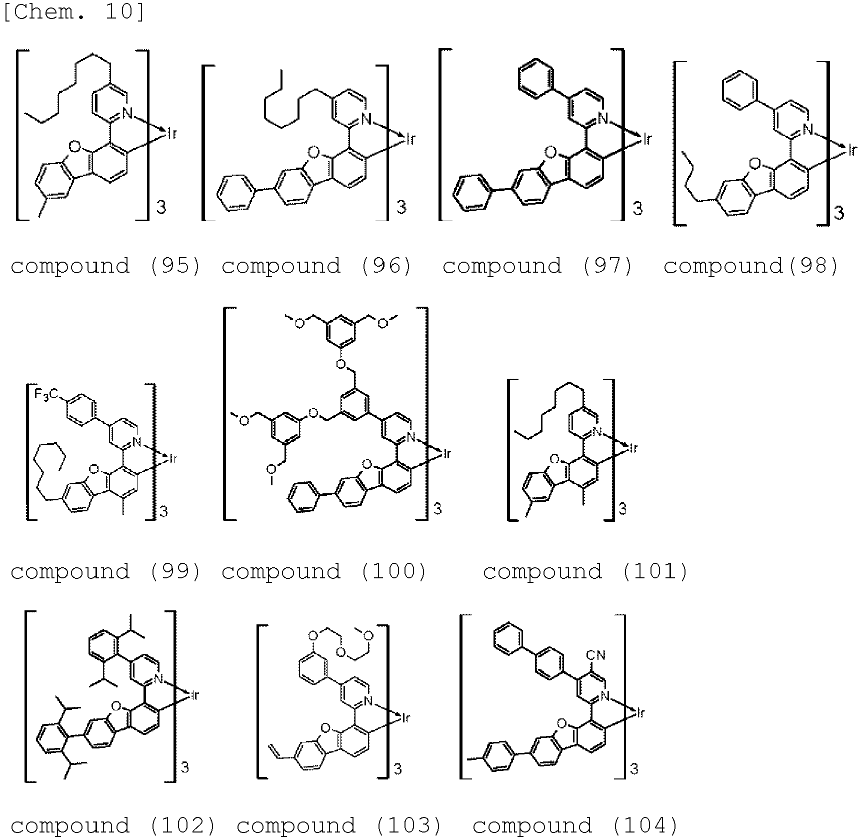

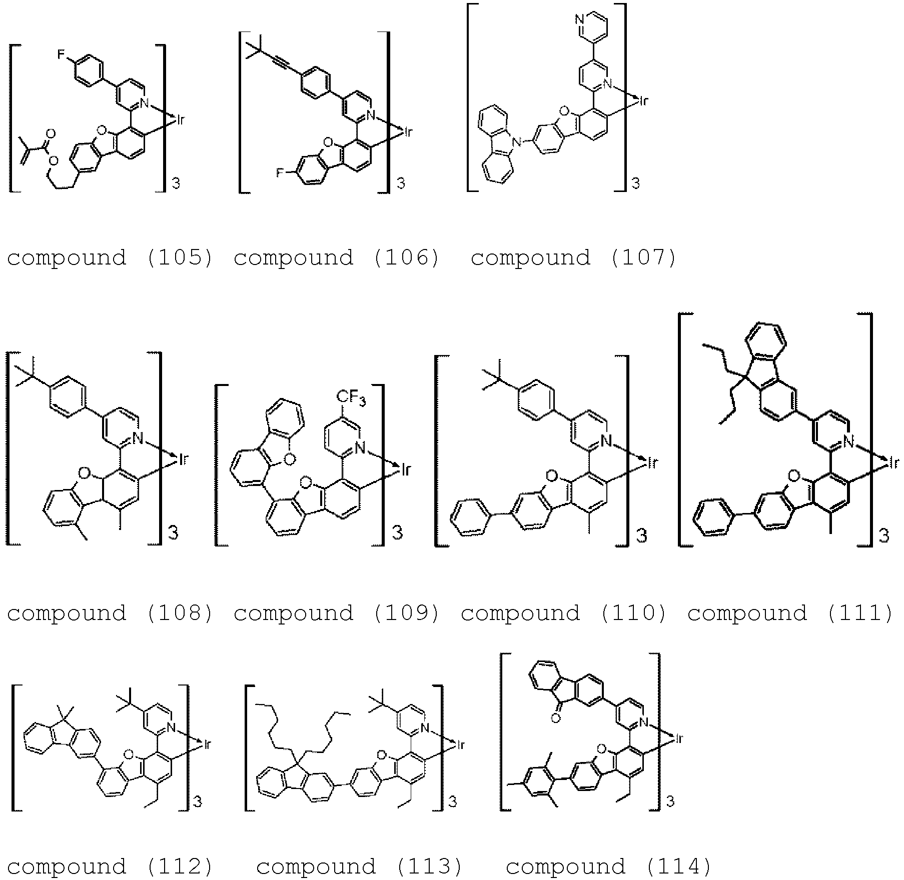

### [Organic Compound Layer Included in Organic Light-Emitting Device According to Embodiment of the Present Invention]

An organic compound layer included in an organic light-emitting device according to an embodiment of the present invention will be described below. The organic light-emitting device according to the present embodiment includes at least a first electrode and a second electrode, which are a pair of electrodes, and the organic compound layer disposed between these electrodes. In the organic light-emitting device according to the present embodiment, the organic compound layer may be formed of a single layer or a laminate including multiple layers, as long as it includes a light-emitting layer. The pair of electrodes may be an anode and a cathode.

When the organic compound layer is formed of a laminate including multiple layers, the organic compound layer may include a light-emitting layer. The organic compound layer may include, in addition to the light-emitting layer, a hole injection layer, a hole transport layer, an electron-blocking layer, a hole-exciton-blocking layer, an electron transport layer, and an electron injection layer, for example. The light-emitting layer may be formed of a single layer or a laminate including multiple layers. The hole transport layer and the electron transport layer are also referred to as charge transport layers.

In the organic light-emitting device according to the present embodiment, at least one layer of the organic compound layers contains an organometallic complex according to the present embodiment. Specifically, the organometallic complex according to the present embodiment is contained in any of the hole injection layer, the hole transport layer, the electron-blocking layer, the light-emitting layer, the hole-exciton blocking layer, the electron transport layer, the electron injection layer, and the like, and is preferably contained in the light-emitting layer. The transport layers between the first electrode and the light-emitting layer can be collectively referred to as a first charge transport layer. The transport layers between the second electrode and the light-emitting layer can be collectively referred to as a second charge transport layer. That is, it can be said that the light-emitting layer is in contact with the first charge transport layer and in contact with the second charge transport layer.

In the organic light-emitting device according to the present embodiment, when the organometallic complex according to the present embodiment is contained in a light-emitting layer, the light-emitting layer may be a layer composed only of the organometallic complex according to the present embodiment or may be a layer containing, in addition to the organometallic complex according to the present embodiment, a first organic compound and a second organic compound different from the first organic compound. The first organic compound may have a higher lowest excited triplet energy than the lowest excited triplet energy of the iridium complex of the present invention. The lowest excited triplet energy of the second organic compound may be equal to or higher than the lowest excited triplet energy of the organometallic complex of the present invention and equal to or lower than the lowest excited triplet energy of the first organic compound. When the light-emitting layer is a layer containing the first organic compound and the second organic compound, the first organic compound may be a host of the light-emitting layer. The second organic compound may be an assist material. The organometallic complex according to the present invention may be a guest or a dopant.

The host used here refers to a compound having the highest proportion by weight in compounds contained in the light-emitting layer. The guest or dopant refers to a compound that has a lower proportion by weight than the host in the compounds contained in the light-emitting layer and that is responsible for main light emission. The assist material refers to a compound that has a lower proportion by weight than the host in the compounds contained in the light-emitting layer and that assists the light emission of the guest. The assist material is also referred to as a second host.

When the organometallic complex according to the present embodiment is used as a guest in the light-emitting layer, the concentration of the guest is preferably 0.01% or more by weight and 20% or less by weight, more preferably 0.1% or more by weight and 10.0% or less by weight, based on the entire light-emitting layer. The entire light-emitting layer refers to the total weight of the compounds constituting the light-emitting layer.

The lowest excited triplet energy of the first charge transport layer is preferably higher than the lowest excited triplet energy of the first organic compound. The lowest excited triplet energy of the second charge transport layer is preferably higher than the lowest excited triplet energy of the first organic compound. The lowest excited triplet energy of the charge transport layer can be estimated by the lowest excited triplet energy of the component material of the layer. When the charge transport layer is composed of a plurality of materials, the lowest excited triplet energy may be the lowest excited triplet energy of a compound having a large proportion by weight.

The inventors have conducted various studies and have found that when the organometallic complex according to the present embodiment is used as a guest of a light-emitting layer, light output with high efficiency and high luminance is exhibited, and good roll-off characteristics are provided. This light-emitting layer can be formed of a single layer or multiple layers and can also contain a light-emitting material having another emission color in order to conduct the color mixture of the emission color of the present embodiment and the other emission color. The term "multiple layers" refers to a state in which multiple light-emitting layers are stacked. In this case, the emission color of the organic light-emitting device is not limited to the same hue as the emission color of the single layer. More specifically, the emission color may be white or an intermediate color. In the case of white light, red light, blue light, and green light may be emitted from the light-emitting layers to obtain white light, or a combination of complementary emission colors may be used to obtain white light.

The organometallic complex according to the present embodiment can be used as a component material of an organic compound layer other than the light-emitting layer included in the organic light-emitting device according to the embodiment. Specifically, the organometallic complex may be used as a component material of the electron transport layer, the electron injection layer, the hole transport layer, the hole injection layer, the hole-blocking layer, and so forth.

When the organic light-emitting device according to the present embodiment is produced, for example, a conventionally known low-molecular-weight or high-molecular-weight hole injection compound, hole transport compound, compound serving as a host, light-emitting compound, electron injection compound, or electron transport compound can be used together as needed. Examples of these compounds are described below.

As a hole injection/transport material, a material having a high hole mobility is preferred in order to facilitate the injection of holes from the anode and to transport the injected holes to the light-emitting layer. To reduce a deterioration in film quality, such as crystallization, in the organic light-emitting device, a material having a high glass transition temperature is preferred. Examples of the low-molecular-weight or high-molecular-weight material having hole injection/transport performance include triarylamine derivatives, aryl carbazole derivatives, phenylenediamine derivatives, triazole derivatives, oxadiazole derivatives, imidazole derivatives, stilbene derivatives, phthalocyanine derivatives, porphyrin derivatives, conductive polymers, such as polyarylamine derivatives, poly(vinyl carbazole) derivatives, polythiophene derivatives, and PEDOT-PSS, copolymers thereof, and mixtures thereof. Moreover, the hole injection/transport material can also be used for the electron-blocking layer.

Specific examples of a compound used as the hole injection/transport material are illustrated below, but of course, hole injection/transport material is not limited thereto.

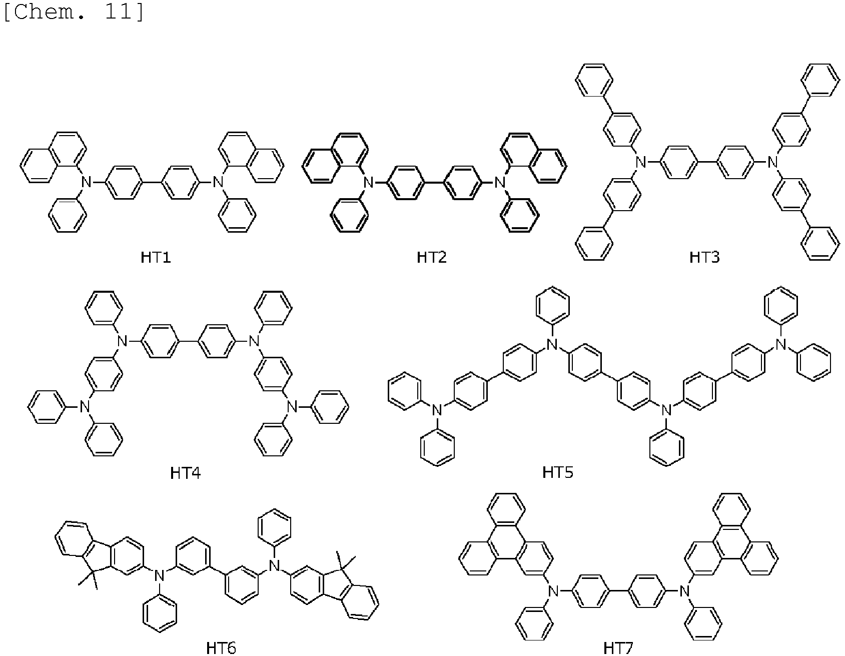

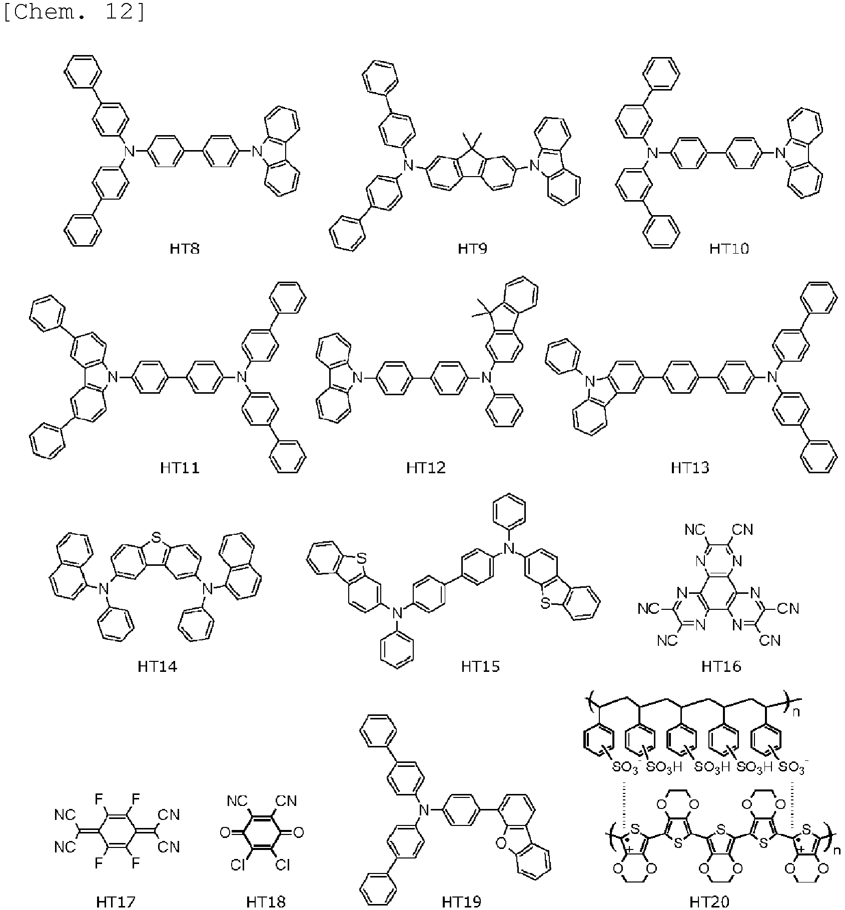

As the light-emitting material mainly associated with a light-emitting function, another light-emitting material can be added in addition to the organometallic complex according to an embodiment of the present invention. Examples of the other light-emitting material include fused-ring compounds, such as fluorene derivatives, naphthalene derivatives, pyrene derivatives, perylene derivatives, tetracene derivatives, anthracene compounds, and rubrene, quinacridone derivatives, coumarin derivatives, stilbene derivatives, organoaluminum complexes, such as tris(8-quinolinolato)aluminum, iridium complexes, such as tris(2-phenylpyridinato)iridium, platinum complexes, rhenium complexes, copper complexes, europium complexes, ruthenium complexes, and polymer derivatives, such as poly(phenylene vinylene) derivatives, polyfluorene derivatives, and polyphenylene derivatives.

Specific examples of a compound used as the light-emitting material are illustrated below, but of course, the light-emitting material is not limited thereto.

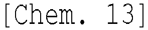

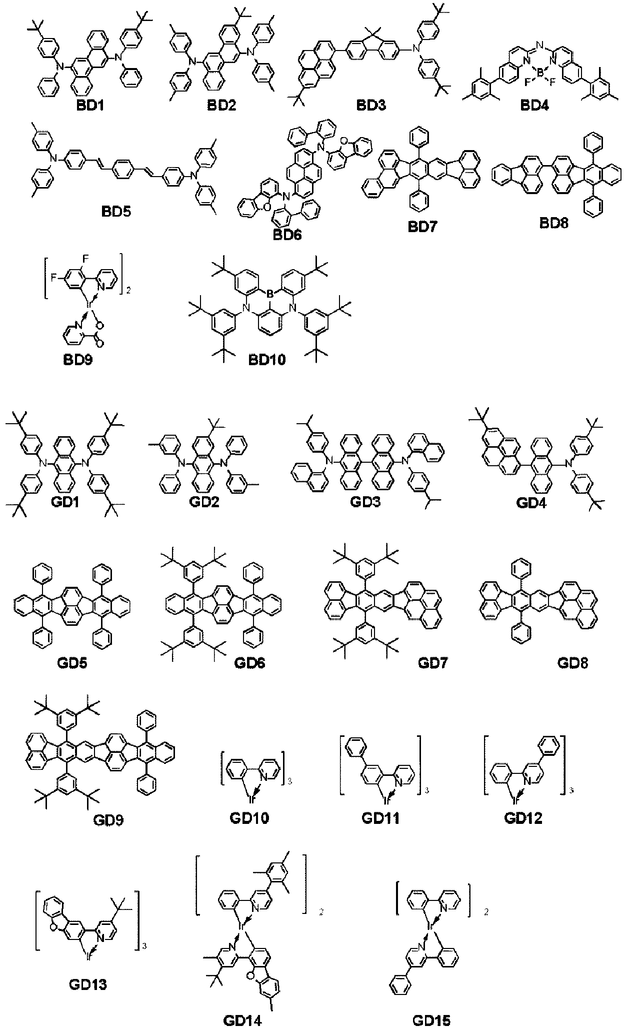

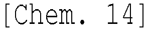

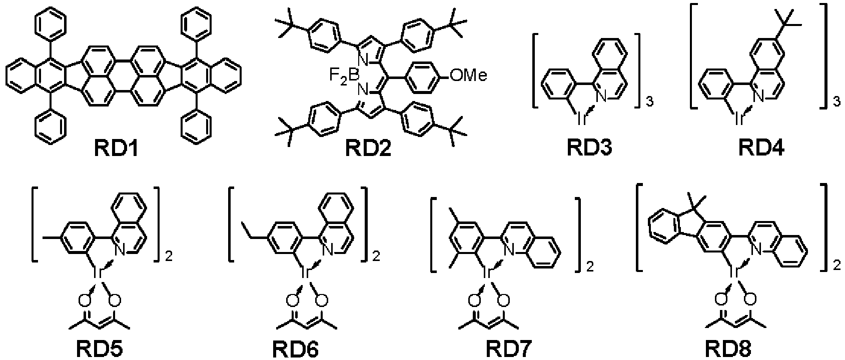

Examples of a light-emitting layer host or a light-emission assist material contained in the light-emitting layer include aromatic hydrocarbon compounds and derivatives thereof, carbazole derivatives, dibenzofuran derivatives, dibenzothiophene derivatives, triazine derivatives, organoaluminum complexes, such as tris(8-quinolinolato)aluminum, organoberyllium complexes, polymers, such as polyphenylene derivatives, poly(phenylene vinylene) derivatives, polyfluorene derivatives, and poly(vinyl carbazole) derivatives, copolymers thereof, and mixtures thereof.

Specific examples of a compound used as the light-emitting layer host or light-emission assist material contained in the light-emitting layer are illustrated below, but of course, the light-emitting layer host or light-emission assist material is not limited thereto.

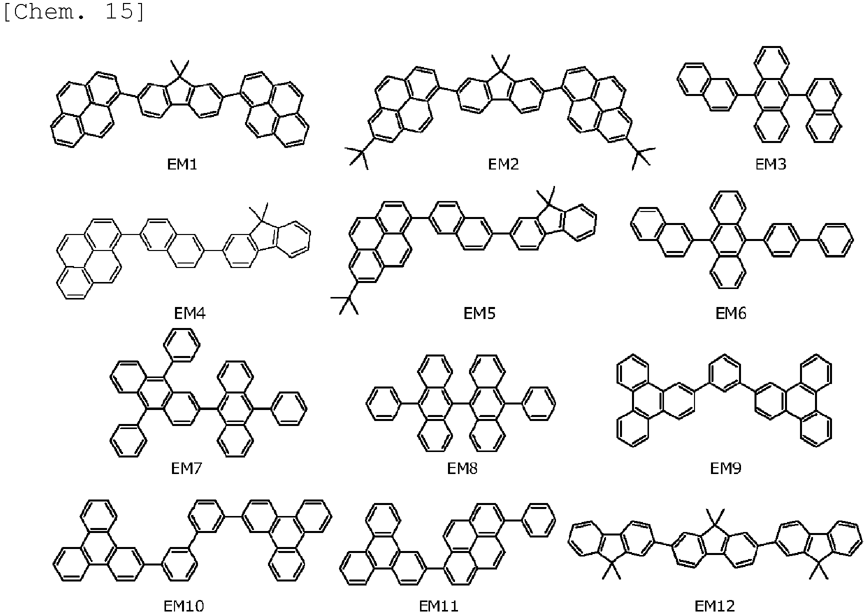

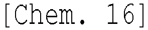

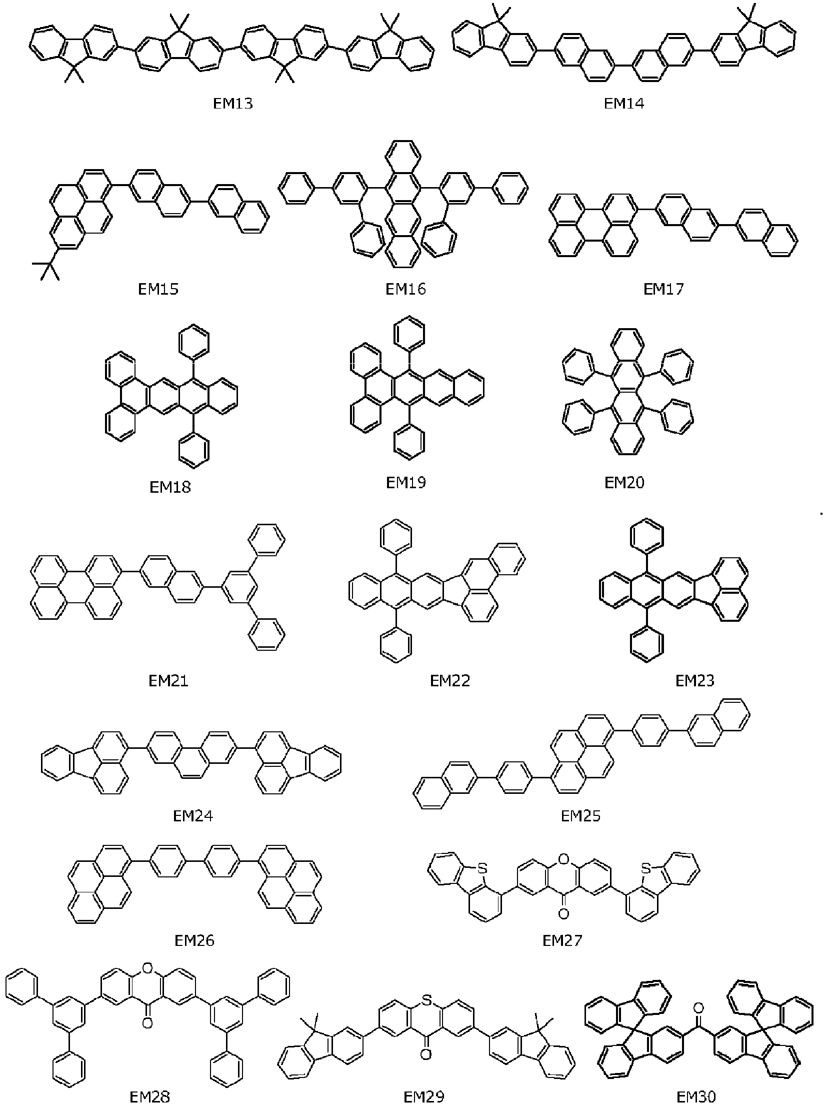

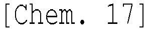

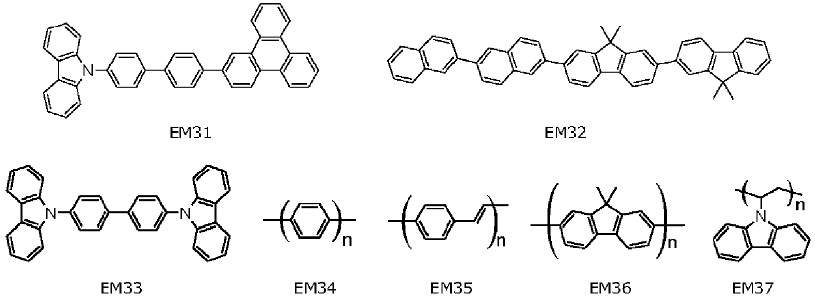

The electron transport material can be freely-selected from materials capable of transporting electrons injected from the cathode to the light-emitting layer and is selected in consideration of, for example, the balance with the hole mobility of the hole transport material. Examples of a material having the ability to transport electrons include oxadiazole derivatives, oxazole derivatives, pyrazine derivatives, triazole derivatives, triazine derivatives, quinoline derivatives, quinoxaline derivatives, phenanthroline derivatives, organoaluminum complexes, and fused-ring compounds (such as fluorene derivatives, naphthalene derivatives, chrysene derivatives, and anthracene derivatives). The electron transport materials are also preferably used for the hole-blocking layer.

Specific examples of a compound used as the electron transport material are illustrated below, but of course, the electron transport material is not limited thereto.

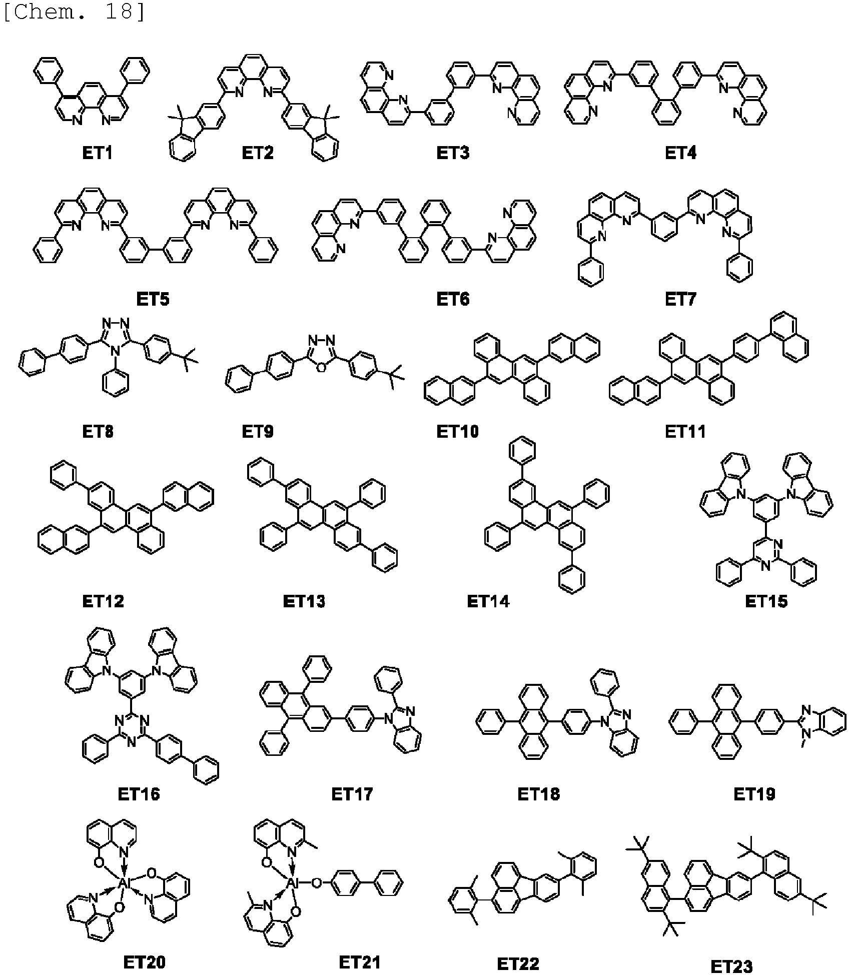

An electron injection material can be freely-selected from materials capable of easily injecting electrons from the cathode and is selected in consideration of, for example, the balance with the hole injection properties. As the organic compound, n-type dopants and reducing dopants are also included. Examples thereof include alkali metal-containing compounds, such as lithium fluoride, lithium complexes, such as lithium quinolinolate, benzimidazolidene derivatives, imidazolidene derivatives, fulvalene derivatives, and acridine derivatives.

### [Configuration of Organic Light-Emitting Device]

The organic light-emitting device is provided by forming a first electrode, an organic compound layer, and a second electrode on an insulating layer provided on a substrate. A protective layer, a color filter, and so forth may be provided on the second electrode. When the color filter is provided, a planarization layer may be provided between the color filter and the protective layer. The planarization layer can be composed of, for example, an acrylic resin. One of the first electrode and the second electrode may be an anode, and the other may be a cathode.

### [Substrate]

Examples of the substrate include quartz, glass, silicon wafers, resins, and metals. The substrate may include a switching element, such as a transistor, a line, and an insulating layer thereon. As the insulating layer, any material can be used as long as a contact hole can be formed to establish the electrical connection between the anode and the line and as long as insulation with a non-connected line can be ensured. For example, a resin, such as polyimide, silicon oxide, or silicon nitride, can be used.

### [Electrode]

A pair of electrodes can be used for the electrodes. The pair of electrodes may be an anode and a cathode. When an electric field is applied in the direction in which the organic light-emitting device emits light, an electrode having a higher potential is the anode, and the other is the cathode. It can also be said that the electrode that supplies holes to the light-emitting layer is the anode and that the electrode that supplies electrons is the cathode.

As the component material of the anode, a material having a work function as high as possible is preferred. Examples of the material that can be used include elemental metals, such as gold, platinum, silver, copper, nickel, palladium, cobalt, selenium, vanadium, and tungsten, mixtures thereof, alloys of combinations thereof, and metal oxides, such as tin oxide, zinc oxide, indium oxide, indium-tin oxide (ITO), and indium-zinc oxide. Additionally, conductive polymers, such as polyaniline, polypyrrole, and polythiophene, can be used.

These electrode materials may be used alone or in combination of two or more. The anode may be formed of a single layer or multiple layers.

When the anode is used as a reflective electrode, for example, chromium, aluminum, silver, titanium, tungsten, molybdenum, an alloy thereof, or a stack thereof can be used. When the anode is used as a transparent electrode, a transparent conductive oxide layer composed of, for example, indium-tin oxide (ITO) or indium-zinc oxide may be used; however, the anode is not limited thereto. The electrode can be formed by photolithography.

As the component material of the cathode, a material having a lower work function is preferred. Examples thereof include elemental metals such as alkali metals, e.g., lithium, alkaline-earth metals, e.g., calcium, aluminum, titanium, manganese, silver, lead, and chromium, and mixtures thereof. Alloys of combinations of these elemental metals can also be used. For example, magnesium-silver, aluminum-lithium, aluminum-magnesium, silver-copper, and zinc-silver can be used. Metal oxides, such as indium-tin oxide (ITO), can also be used. These electrode materials may be used alone or in combination of two or more. The cathode may have a single-layer structure or a multilayer structure. Among these, silver is preferably used, and a silver alloy is more preferably used in order to inhibit aggregation of silver. The alloy ratio is not limited as long as the aggregation of silver can be inhibited. For example, it may be 1:1.

A top emission device may be provided using the cathode formed of a conductive oxide layer composed of, for example, indium-tin oxide (ITO). A bottom emission device may be provided using the cathode formed of a reflective electrode composed of, for example, aluminum (Al). Any type of cathode may be used. A method for forming the cathode is not particularly limited, but a direct-current sputtering method, an alternating-current sputtering method, or the like is more preferably used because good film coverage is obtained and thus the resistance is easily reduced.

### [Protective Layer]

A protective layer may be disposed on the cathode. For example, a glass member provided with a moisture absorbent can be bonded to the cathode to reduce the entry of, for example, water into the organic compound layer, thereby reducing the occurrence of display defects. In another embodiment, a passivation film composed of, for example, silicon nitride may be disposed on the cathode to reduce the entry of, for example, water into the organic compound layer. For example, after the formation of the cathode, the substrate may be transported to another chamber without breaking the vacuum, and a silicon nitride film having a thickness of 2 µm may be formed by a CVD method to provide a protective layer. After the film deposition by the CVD method, a protective layer may be formed by an atomic layer deposition (ALD) method.

### [Color Filter]

A color filter may be disposed on the protective layer. For example, a color filter may be disposed on another substrate in consideration of the size of the organic light-emitting device and bonded to the substrate provided with the organic light-emitting device. A color filter may be formed by patterning on the protective layer using photolithography. The color filter may be composed of a polymer.

### [Planarization Layer]

A planarization layer may be disposed between the color filter and the protective layer. The planarization layer may be composed of an organic compound. A low- or high-molecular-weight organic compound may be used. A high-molecular-weight organic compound is preferred.

The planarization layers may be disposed above and below (or on) the color filter and may be composed of the same or different component materials. Specific examples thereof include poly(vinyl carbazole) resins, polycarbonate resins, polyester resins, ABS resins, acrylic resins, polyimide resins, phenolic resins, epoxy resins, silicon resins, and urea resins.

### [Opposite Substrate]

An opposite substrate may be disposed on the planarization layer. The opposite substrate is disposed at a position corresponding to the substrate described above and thus is called an opposite substrate. The opposite substrate may be composed of the same material as the substrate described above. When the above-described substrate is referred to as a first substrate, the opposite substrate may be referred to as a second substrate.

### [Formation of Organic Compound Layer]

The organic compound layer (such as the hole injection layer, the hole transport layer, the electron-blocking layer, the light-emitting layer, the hole-blocking layer, the electron transport layer, or the electron injection layer) included in the organic light-emitting device according to an embodiment of the present invention is formed by a method described below.

The organic compound layer constituting the organic light-emitting device according to an embodiment of the present invention can be formed by a dry process or a wet process without any particular limitation. Examples of the dry process that can be used include a vacuum evaporation method, an ionized evaporation method, sputtering, and plasma. Examples of the wet process include known coating methods (for example, a spin coating method, a casting method, a microgravure coating method, a gravure coating method, a bar coating method, a roll coating method, a wire bar coating method, a dip coating method, a spray coating method, a screen printing method, a flexographic printing method, an offset printing method, an ink jet printing method, a capillary coating method, and a nozzle coating method) using a solution prepared by dissolving the compound in an appropriate solvent. Among these, a vacuum evaporation method, an ionized evaporation method, an ink jet printing method, a nozzle coating method, and the like are suitable for manufacturing an organic light-emitting device having a large area.

The thickness of each of the layers in the organic light-emitting device is usually preferably 1 nm to 10 µm. In particular, the thickness of the light-emitting layer of the organic compound layer is preferably 10 nm to 100 nm in order to obtain effective light emission characteristics.

When the light-emitting layer of the organic compound layer is formed by a wet process, the compositions of these layers is dissolved in a solvent to form inks. The viscosity of each ink may be adjusted in accordance with the type of printing method. When such an ink is used in a printing method, such as an ink jet printing method, in which a solution passes through a discharge device, the viscosity is preferably 1 to 20 mPa·s at 25°C in order to reduce clogging and flight deflection during discharge.

Usually, a solvent having a boiling point of 70°C to 300°C at 1 atm can be used for the ink. The amount of organic solvent is usually 10 to 100 parts by mass based on 1 part by mass of a material constituting each organic compound layer.

Drying of the coating film obtained by the wet process can be appropriately selected in accordance with the type of each layer. Usually, the heating can be performed at 100°C to 250°C, preferably 110°C to 200°C, for 5 to 60 minutes in an air atmosphere or an inert gas (nitrogen, argon, etc.) atmosphere. Alternatively, heating may be performed under normal pressure (1 atm) or reduced pressure (100 Pa to 0.1 MPa). The temperature, pressure, and time in the drying step can be adjusted to remove the solvent in each layer.

In the case of forming a film by the coating method, the film may be formed in combination with an appropriate binder resin.

Examples of the binder resin include, but are not limited to, poly(vinyl carbazole) resins, polycarbonate resins, polyester resins, ABS resins, acrylic resins, polyimide resins, phenolic resins, epoxy resins, silicon resins, and urea resins.

These binder resins may be used alone as a homopolymer or copolymer, or in combination as a mixture of two or more. Furthermore, additives, such as a known plasticizer, antioxidant, and ultraviolet absorber, may be used, as needed.

### [Pixel Circuit]

A light-emitting apparatus may include pixel circuits coupled to the light-emitting devices. Each of the pixel circuits may be of an active matrix type, which independently controls the emission of first and second light-emitting devices. The active matrix-type circuit may be voltage programming or current programming. A driving circuit includes the pixel circuit for each pixel. The pixel circuit may include a light-emitting device, a transistor for controlling the luminance of the light-emitting device, a transistor for controlling the timing of the light emission, a capacitor for retaining the gate voltage of the transistor for controlling the luminance, and a transistor for connecting to GND without using the light-emitting device.

The light-emitting apparatus includes a display area and a peripheral area disposed around the display area. The display area includes a pixel circuit, and the peripheral area includes a display control circuit. The mobility of a transistor contained in the pixel circuit may be lower than the mobility of a transistor contained in the display control circuit.

The slope of the current-voltage characteristics of the transistor contained in the pixel circuit may be smaller than the slope of the current-voltage characteristic of the transistor contained in the display control circuit. The slope of the current-voltage characteristics can be measured by what is called Vg-Ig characteristics.

The transistor contained in the pixel circuit is a transistor coupled to a light-emitting device, such as a first light-emitting device.

### [Pixel]

The organic light-emitting apparatus includes multiple pixels. Each pixel includes subpixels configured to emit colors different from each other. The subpixels may have respective RGB emission colors.

Light emerges from a region of the pixel, also called a pixel aperture. This region is the same as a first region. The pixel aperture may be 15 µm or less, and may be 5 µm or more. More specifically, the pixel aperture may be, for example, 11 pm, 9.5 pm, 7.4 pm, or 6.4 µm.

The distance between subpixels may be 10 µm. Specifically, the distance may be 8 pm, 7.4 pm, or 6.4 µm.

The pixels may be arranged in a known pattern in plan view. For example, a stripe pattern, a delta pattern, a Pen Tile matrix pattern, or the Bayer pattern may be used. The shape of each subpixel in plan view may be any known shape. Examples of the shape of the subpixel include quadrilaterals, such as rectangles and rhombi, and hexagons. Of course, if the shape is close to a rectangle, rather than an exact shape, it is included in the rectangle. The shape of the subpixel and the pixel arrangement can be used in combination.

### [Application of Organic Light-Emitting Device According to an Embodiment of The Present Invention]

The organic light-emitting device according to an embodiment of the present invention can be used as a component member of a display apparatus or lighting apparatus. Other applications include exposure light sources for electrophotographic image-forming apparatuses, backlights for liquid crystal display apparatuses, and light-emitting apparatuses including white-light sources and color filters.

The display apparatus may be an image information-processing unit having an image input unit that receives image information from an area or linear CCD sensor, a memory card, or any other source, an information-processing unit that processes the input information, and a display unit that displays the input image.

The display unit of an image pickup apparatus or an inkjet printer may have a touch panel function. The driving mode of the touch panel function may be, but is not particularly limited to, an infrared mode, an electrostatic capacitance mode, a resistive film mode, or an electromagnetic inductive mode. The display apparatus may also be used for a display unit of a multifunction printer.

The following describes a display apparatus according to the present embodiment with reference to the attached drawings.

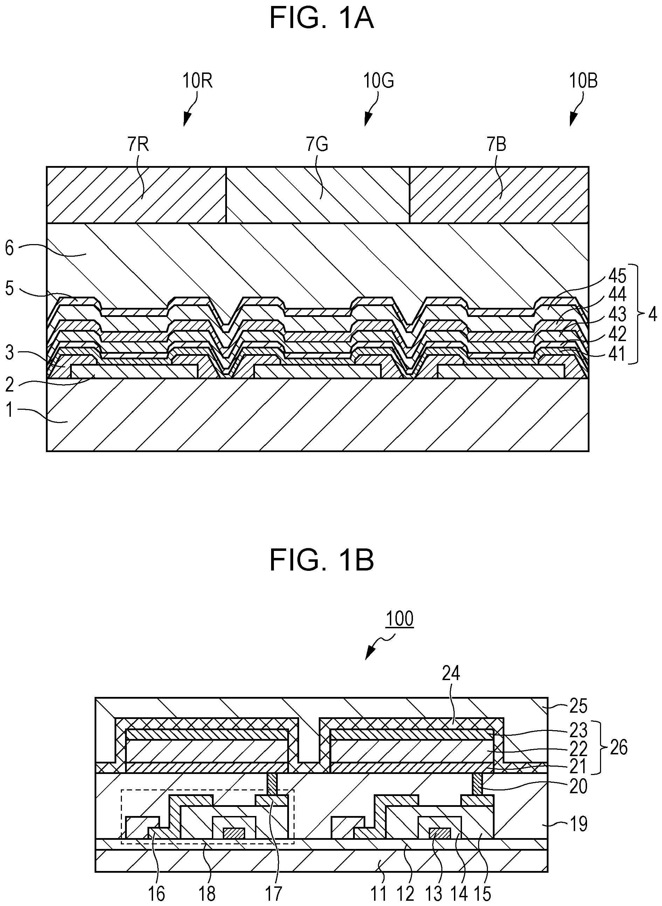

Fig. 1A is a schematic cross-sectional view of an example of a pixel of a display apparatus according to the present embodiment. Each of the pixels includes subpixels 10. The subpixels are divided into 10R, 10G, and 10B according to their light emission. The emission colors may be distinguished by the wavelength of light emitted from the light-emitting layer. Light emitted from the subpixels may be selectively transmitted or color-converted with, for example, a color filter. Each subpixel includes a reflective electrode 2 serving as a first electrode, an insulating layer 3 covering the edge of the reflective electrode 2, an organic compound layer 4 covering the first electrode and the insulating layer, a transparent electrode 5, a protective layer 6, and a color filter 7, over an interlayer insulating layer 1.

The transistors and capacitive elements may be disposed under or in the interlayer insulating layer 1. Each transistor may be electrically coupled to a corresponding one of the first electrodes through a contact hole (not illustrated) .

The insulating layer 3 is also called a bank or pixel separation film. The insulating layer covers the edge of each first electrode and surrounds the first electrode. Portions that are not covered with the insulating layer are in contact with the organic compound layer 4 and serve as light-emitting regions.

The organic compound layer 4 includes a hole injection layer 41, a hole transport layer 42, a first light-emitting layer 43, a second light-emitting layer 44, and an electron transport layer 45. The second electrode 5 may be a transparent electrode, a reflective electrode, or a semitransparent electrode.

The protective layer 6 reduces the penetration of moisture into the organic compound layer. Although the protective layer is illustrated as a single layer, the protective layer may include multiple layers, and each layer may be an inorganic compound layer or an organic compound layer.

The color filter 7 is separated into 7R, 7G, and 7B according to its color. The color filter may be disposed on a planarization film (not illustrated). A resin protective layer (not illustrated) may be disposed on the color filter. The color filter may be disposed on the protective layer 6. Alternatively, the color filter may be disposed on an opposite substrate, such as a glass substrate, and then bonded.

Fig. 1B is a schematic cross-sectional view of an example of a display apparatus including organic light-emitting devices and transistors coupled to the respective organic light-emitting devices. Each of the transistors is an example of an active element. The transistors may be thin-film transistors (TFTs).

A display apparatus 100 illustrated in Fig. 1B includes a substrate 11 composed of, for example, glass or silicon, and an insulating layer 12 provided thereon. Active elements, such as TFTs 18, are arranged on the insulating layer. A gate electrode 13, a gate insulating film 14, and a semiconductor layer 15 of each of the active elements are arranged. Each TFT 18 includes the semiconductor layer 15, a drain electrode 16, and a source electrode 17. An insulating film 19 is disposed on the TFTs 18. An anode 21 included in the organic light-emitting devices is coupled to the source electrodes 17 through contact holes 20 provided in the insulating film.

The mode of electrical connection between the electrodes (anode and cathode) included in each organic light-emitting device and the electrodes (source electrode and drain electrode) included in a corresponding one of the TFTs is not limited to the mode illustrated in Fig. 1B. That is, it is sufficient that any one of the anode and the cathode is electrically coupled to any one of the source electrode and the drain electrode of the TFT. The term "TFT" refers to a thin-film transistor.

In the display apparatus 100 illustrated in Fig. 1B, each organic compound layer is illustrated as a single layer; however, the organic compound layer 22 may be formed of multiple layers. To reduce the deterioration of the organic light-emitting devices, a first protective layer 24 and a second protective layer 25 are disposed on the cathodes 23.

In the display apparatus 100 illustrated in Fig. 1B, although the transistors are used as switching elements, other switching elements may be used instead.

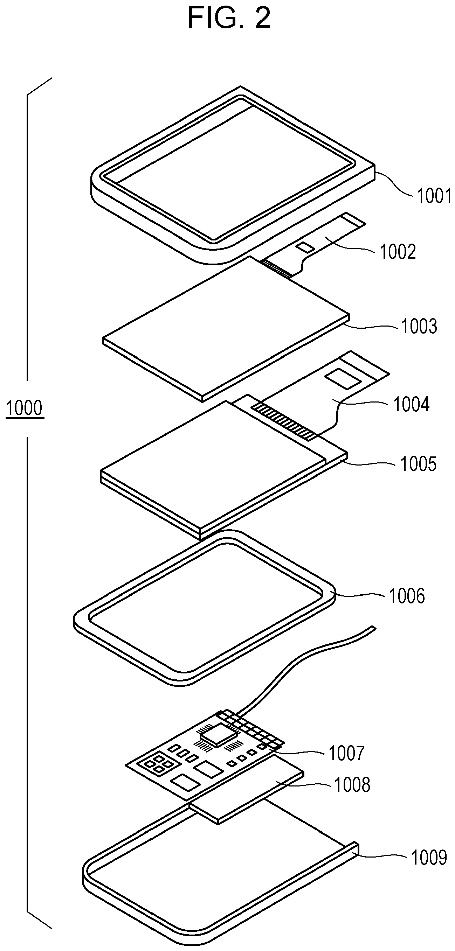

The transistors used in the display apparatus 100 illustrated in Fig. 2(b) are not limited to transistors using a single-crystal silicon wafer, but may also be thin-film transistors including active layers on the insulating surface of a substrate. Examples of the material of the active layer include single-crystal silicon, non-single-crystal silicon materials, such as amorphous silicon and microcrystalline silicon, and non-single-crystal oxide semiconductors, such as indium-zinc oxide and indium-gallium-zinc oxide. Thin-film transistors are also called TFT elements.

The transistors in the display apparatus 100 illustrated in Fig. 1B may be formed in the substrate, such as a Si substrate. The expression "formed in the substrate" indicates that the transistors are produced by processing the substrate such as a Si substrate. When the transistors are formed in the substrate, the substrate and the transistors can be deemed to be integrally formed.

In the organic light-emitting device according to the present embodiment, the luminance is controlled by the TFTs, which are an example of switching elements; thus, an image can be displayed at respective luminance levels by arranging multiple organic light-emitting devices in the plane. The switching elements according to the present embodiment are not limited to the TFT elements and may be low-temperature polysilicon transistors or active-matrix drivers formed on a substrate such as a Si substrate. The expression "on a substrate" can also be said to be "in the substrate". Whether transistors are formed in the substrate or TFTs are used is selected in accordance with the size of a display unit. For example, when the display unit has a size of about 0.5 inches, organic light-emitting devices are preferably disposed on a Si substrate.

Fig. 2 is a schematic view illustrating an example of a display apparatus according to the present embodiment. A display apparatus 1000 may include a touch panel 1003, a display panel 1005, a frame 1006, a circuit board 1007, and a battery 1008 disposed between an upper cover 1001 and a lower cover 1009. The touch panel 1003 and the display panel 1005 are coupled to flexible printed circuits FPCs 1002 and 1004, respectively. The circuit board 1007 includes printed transistors. The battery 1008 need not be provided unless the display apparatus is a portable apparatus. The battery 1008 may be disposed at a different position even if the display apparatus is a portable apparatus.

The display apparatus according to the present embodiment may include a color filter having red, green, and blue portions. In the color filter, the red, green, and blue colors may be arranged in a delta arrangement, a stripe arrangement, or a mosaic arrangement.

The display apparatus according to the present embodiment may be used for the display unit of a portable terminal. In that case, the display apparatus may have both a display function and an operation function. Examples of the portable terminal include cellular phones, such as smartphones, tablets, and head-mounted displays.

The display apparatus according to the present embodiment may be used for a display unit of an image pickup apparatus including an optical unit including multiple lenses and an image pickup device that receives light passing through the optical unit. The image pickup apparatus may include a display unit that displays information acquired by the image pickup device. The display unit may be a display unit exposed to the outside of the image pickup apparatus or a display unit disposed in a finder. The image pickup apparatus may be a digital camera or a digital camcorder. The image pickup apparatus can be also referred to as a photoelectric conversion apparatus.

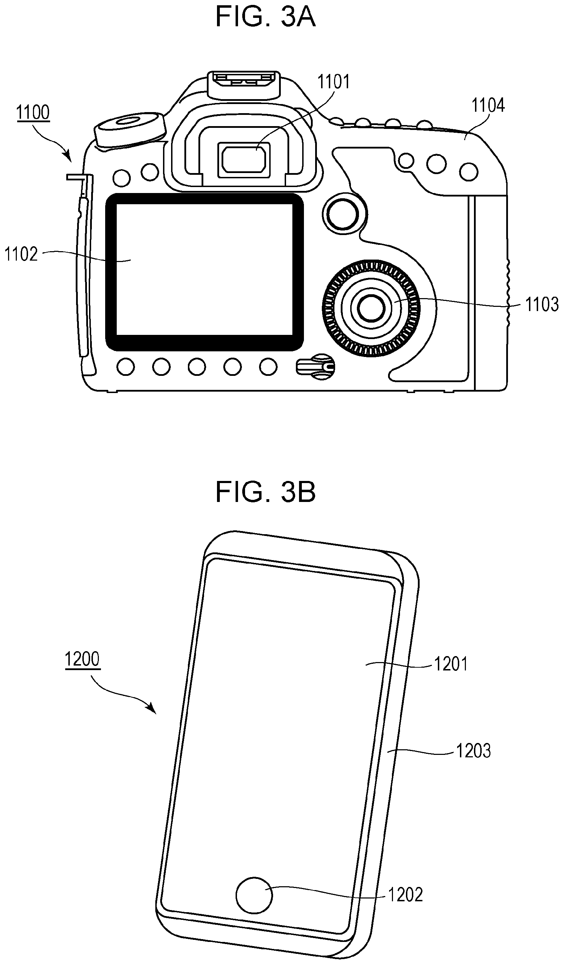

Fig. 3A is a schematic view illustrating an example of an image pickup apparatus according to the present embodiment. An image pickup apparatus 1100 may include a viewfinder 1101, a rear display 1102, an operation unit 1103, and a housing 1104. The viewfinder 1101 may include the display apparatus according to the present embodiment. In this case, the display apparatus may display environmental information, imaging instructions, and so forth in addition to an image to be captured. The environmental information may include, for example, the intensity of external light, the direction of the external light, the moving speed of a subject, and the possibility that a subject is shielded by a shielding material.

The timing suitable for imaging is only for a short time; thus, the information may be displayed as soon as possible. Thus, a display apparatus including the organic light-emitting device according to the present invention is preferably used. This is because the organic light-emitting device has a high response speed. The display apparatus including the organic light-emitting device can be used more suitably than liquid crystal display apparatuses for such apparatuses required to have a high display speed.

The image pickup apparatus 1100 includes an optical unit (not illustrated). The optical unit includes multiple lenses and is configured to form an image on an image pickup device in the housing 1104. The relative positions of the multiple lenses can be adjusted to adjust the focal point. This operation can also be performed automatically.

Fig. 3B is a schematic view illustrating an example of an electronic apparatus according to the present embodiment. An electronic apparatus 1200 includes a display unit 1201, an operation unit 1202, and a housing 1203. The housing 1203 may accommodate a circuit, a printed circuit board including the circuit, a battery, and a communication unit. The operation unit 1202 may be a button or a touch-panel-type reactive unit. The operation unit may be a biometric recognition unit that recognizes a fingerprint to release the lock or the like. An electronic apparatus including a communication unit can also be referred to as a communication apparatus. The electronic apparatus may further have a camera function by being equipped with a lens and an image pickup device. An image captured by the camera function is displayed on the display unit. Examples of the electronic apparatus include smartphones and notebook computers.

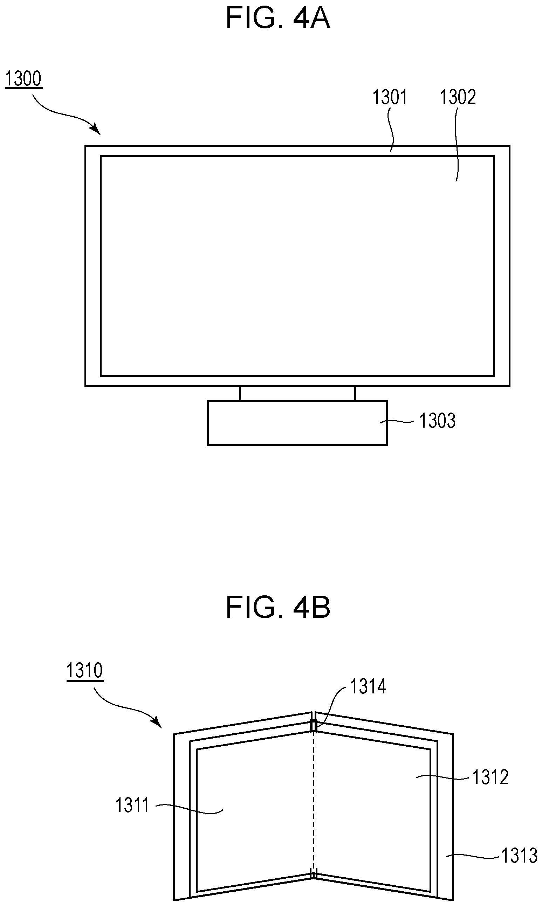

Figs. 4A and 4B are each a schematic view illustrating an example of a display apparatus according to the present embodiment. Fig. 4A illustrates a display apparatus, such as a television monitor or a PC monitor. A display apparatus 1300 includes a frame 1301 and a display unit 1302. The display unit 1302 may include a light-emitting apparatus according to the present embodiment.

A base 1303 that supports the frame 1301 and the display unit 1302 is provided. The base 1303 is not limited to the structure illustrated in Fig. 4A. The lower side of the frame 1301 may also serve as a base.

The frame 1301 and the display unit 1302 may be curved. These may have a radius of curvature of 5,000 mm or more and 6,000 mm or less.

Fig. 4B is a schematic view illustrating another example of a display apparatus according to the present embodiment. A display apparatus 1310 illustrated in Fig. 4B can be folded and is what is called a foldable display apparatus. The display apparatus 1310 includes a first display portion 1311, a second display portion 1312, a housing 1313, and an inflection point 1314. The first display portion 1311 and the second display portion 1312 may include a light-emitting apparatus according to the present embodiment. The first display portion 1311 and the second display portion 1312 may be a single, seamless display apparatus. The first display portion 1311 and the second display portion 1312 can be divided from each other at the inflection point. The first display portion 1311 and the second display portion 1312 may display different images from each other. Alternatively, a single image may be displayed in the first and second display portions.

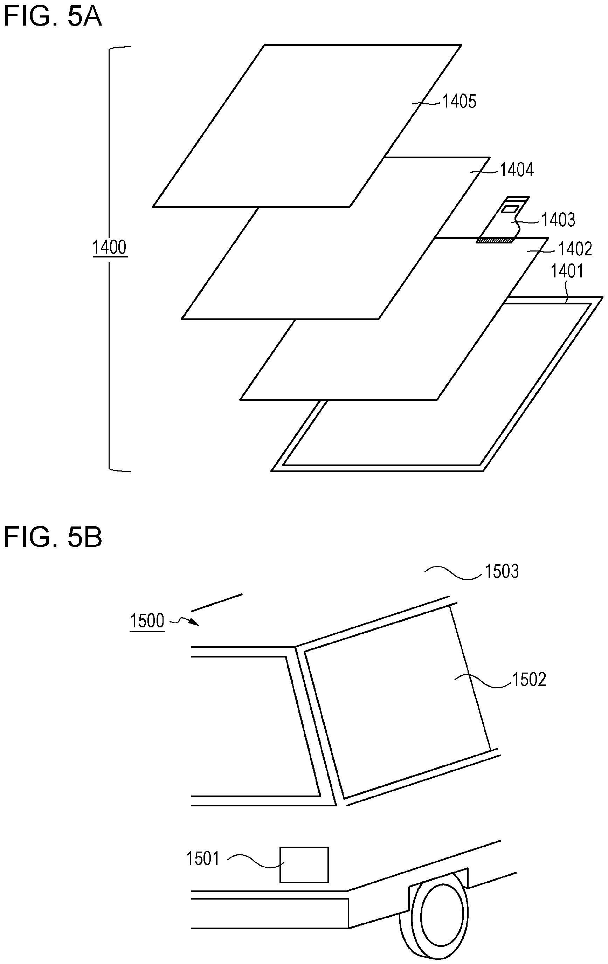

Fig. 5A is a schematic view illustrating an example of a lighting apparatus according to the present embodiment. A lighting apparatus 1400 may include a housing 1401, a light source 1402, a circuit board 1403, an optical film 1404, and a light diffusion unit 1405. The light source may include an organic light-emitting device according to the present embodiment. The optical filter may be a filter that improves the color rendering properties of the light source. The light diffusion unit can effectively diffuse light from the light source to deliver the light to a wide range when used for illumination and so forth. The optical filter and the light diffusion unit may be disposed at the light emission side of the lighting device. A cover may be disposed at the outermost portion, as needed.

The lighting apparatus is, for example, an apparatus that lights a room. The lighting apparatus may emit light of white, neutral white, or any color from blue to red. A light control circuit for controlling the light may be provided. The lighting apparatus may include the organic light-emitting device the present invention and a power supply circuit coupled thereto. The power supply circuit is a circuit that converts an AC voltage into a DC voltage. The color temperature of white is 4,200 K, and the color temperature of neutral white is 5,000 K. The lighting apparatus may include a color filter.

The lighting apparatus according to the present embodiment may include a heat dissipation unit. The heat dissipation unit is configured to release heat in the apparatus to the outside of the apparatus and is composed of, for example, a metal having a high specific heat or liquid silicon.

Fig. 5B is a schematic view illustrating an automobile, as an example of a moving object, according to the present embodiment. The automobile includes a tail lamp, which is an example of lighting units. An automobile 1500 includes a tail lamp 1501 and may be configured to light the tail lamp when a brake operation or the like is performed.

The tail lamp 1501 may include an organic light-emitting device according to the present embodiment. The tail lamp may include a protective member that protects the organic light-emitting device. The protective member may be composed of any transparent material having high strength to some extent and is preferably composed of, for example, polycarbonate. The polycarbonate may be mixed with, for example, a furandicarboxylic acid derivative or an acrylonitrile derivative.

The automobile 1500 may include an automobile body 1503 and windows 1502 attached thereto. The windows may be transparent displays if the windows are not used to check areas ahead and behind of the automobile. The transparent displays may include an organic light-emitting device according to the present embodiment. In this case, the component materials, such as the electrodes, of the organic light-emitting device are formed of transparent members.

The moving object according to the present embodiment may be, for example, a ship, an aircraft, or a drone. The moving object may include a body and a lighting unit attached to the body. The lighting unit may emit light to indicate the position of the body. The lighting unit includes the organic light-emitting device according to the present embodiment.

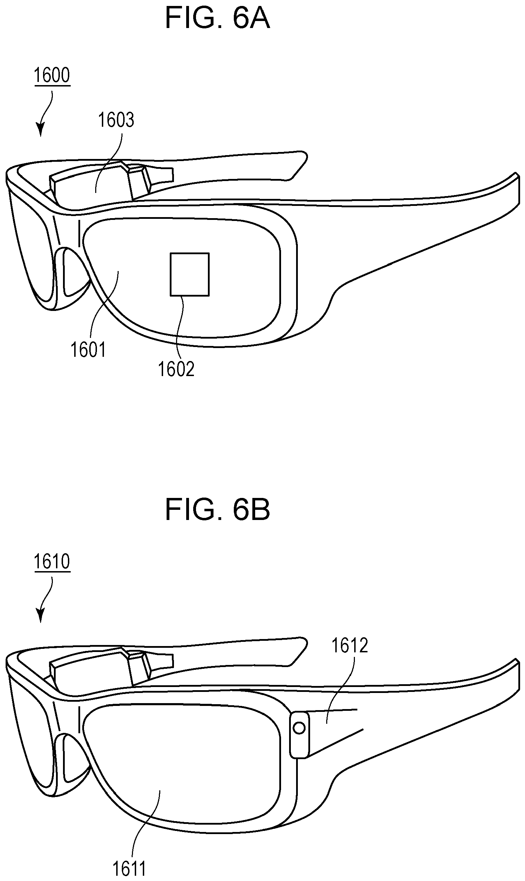

Examples of applications of the display apparatuses of the above embodiments will be described with reference to Figs. 6A and 6B. The display apparatus can be used for systems that can be worn as wearable devices, such as smart glasses, HMDs, and smart contact lenses. An image pickup and display apparatus used in such an application has an image pickup apparatus that can photoelectrically convert visible light and a display apparatus that can emit visible light.

Fig. 6A illustrates glasses 1600 (smart glasses) according to an application example. An image pickup apparatus 1602, such as a CMOS sensor or SPAD, is provided on a front side of a lens 1601 of the glasses 1600. The display apparatus according to any of the above-mentioned embodiments is provided on the back side of the lens 1601.

The glasses 1600 further include a control unit 1603. The control unit 1603 functions as a power source that supplies electric power to the image pickup apparatus 1602 and the display apparatus according to any of the embodiments. The control unit 1603 controls the operation of the image pickup apparatus 1602 and the display apparatus. The lens 1601 has an optical system for focusing light on the image pickup apparatus 1602.

Fig. 6B illustrates glasses 1610 (smart glasses) according to an application example. The glasses 1610 include a control unit 1612. The control unit 1612 includes an image pickup apparatus corresponding to the image pickup apparatus 1602 and a display apparatus. A lens 1611 is provided with the image pickup apparatus in the control unit 1612 and an optical system that projects light emitted from the display apparatus. An image is projected onto the lens 1611. The control unit 1612 functions as a power source that supplies electric power to the image pickup apparatus and the display apparatus and controls the operation of the image pickup apparatus and the display apparatus. The control unit may include a gaze detection unit that detects the gaze of a wearer. Infrared light may be used for gaze detection. An infrared light-emitting unit emits infrared light to an eyeball of a user who is gazing at a displayed image. An image of the eyeball is captured by detecting the reflected infrared light from the eyeball with an image pickup unit having light-receiving elements. The deterioration of image quality is reduced by providing a reduction unit configured to reduce light from the infrared light-emitting unit to the display unit when viewed in plan.

The user's gaze at the displayed image is detected from the image of the eyeball captured with the infrared light. Any known method can be used to the gaze detection using the captured image of the eyeball. As an example, a gaze detection method based on a Purkinje image of the reflection of irradiation light on a cornea can be used.

More specifically, the gaze detection process is performed on the basis of a pupil-corneal reflection method. Using the pupil-corneal reflection method, the user's gaze is detected by calculating a gaze vector representing the direction (rotation angle) of the eyeball based on the image of the pupil and the Purkinje image contained in the captured image of the eyeball.

A display apparatus according to an embodiment of the present invention may include an image pickup apparatus including light-receiving elements, and may control an image displayed on the display apparatus based on the gaze information of the user from the image pickup apparatus.

Specifically, in the display apparatus, a first field-of-view area at which the user gazes and a second field-of-view area other than the first field-of-view area are determined on the basis of the gaze information. The first field-of-view area and the second field-of-view area may be determined by the control unit of the display apparatus or may be determined by receiving those determined by an external control unit. In the display area of the display apparatus, the display resolution of the first field-of-view area may be controlled to be higher than the display resolution of the second field-of-view area. That is, the resolution of the second field-of-view area may be lower than that of the first field-of-view area.

The display area includes a first display area and a second display area different from the first display area. Based on the gaze information, an area of higher priority is determined from the first display area and the second display area. The first field-of-view area and the second field-of-view area may be determined by the control unit of the display apparatus or may be determined by receiving those determined by an external control unit. The resolution of an area of higher priority may be controlled to be higher than the resolution of an area other than the area of higher priority. In other words, the resolution of an area of a relatively low priority may be low.

Artificial intelligence (AI) may be used to determine the first field-of-view and the high-priority area. The AI may be a model configured to estimate the angle of gaze from the image of the eyeball and the distance to a target object located in the gaze direction, using the image of the eyeball and the actual direction of gaze of the eyeball in the image as teaching data. The AI program may be stored in the display apparatus, the image pickup apparatus, or an external apparatus. When the AI program is stored in the external apparatus, the AI program is transmitted to the display apparatus via communications.

In the case of controlling the display based on visual detection, smart glasses that further include an image pickup apparatus that captures an external image can be used. The smart glasses can display the captured external information in real time.

As described above, the use of an apparatus including the organic light-emitting device according to the present embodiment enables a stable display with good image quality even for a long time.

As described above, the use of an apparatus including the organic light-emitting device according to the present embodiment enables both good outdoor visibility and power-saving display due to high efficiency and high luminance light output.

### EXAMPLES

Examples will be described below. However, the present invention is not limited these examples.

### [Synthesis Example 1]

### Synthesis of Compound (4)

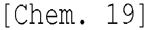

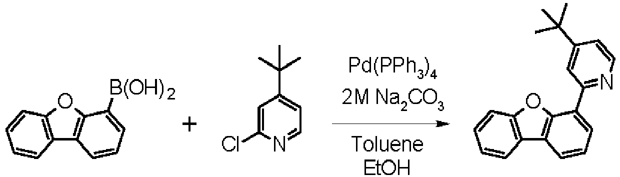

Compound (4) was synthesized by the following procedure. First, synthesis of a ligand will be described. In a nitrogen atmosphere, 2.12 g (10.5 mmol) of dibenzofuran-4-boronic acid, 1.70 g (10.0 mmol) of 4-(tert-butyl)-2-chloropyridine, 0.12 g (0.1 mmol) of tetrakis(triphenylphosphine)palladium, 30 ml of toluene, 15 ml of ethanol, and 15 ml of 2 M aqueous sodium carbonate solution were placed in a 100-ml recovery flask. The mixture was heated from room temperature to 90°C and stirred for 4 hours. Toluene and water were added thereto. The organic layer was extracted. Magnesium sulfate was added to the resulting organic layer, and then filtration was performed. The filtrate was concentrated and purified by silica gel column chromatography (mobile phase: chloroform). The solvent was removed by evaporation, and crystallization was performed using an aqueous solution of isopropyl alcohol (IPA) to give 3.00 g of a ligand. The structure was identified by ¹H MS.

The synthesis of compound (4) will be described below.

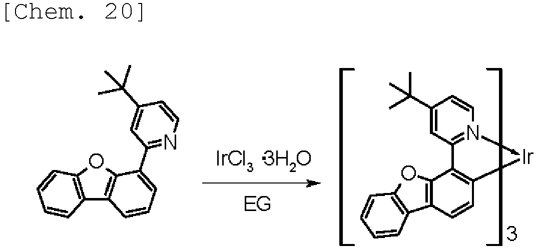

In a nitrogen atmosphere, 70.5 mg (0.2 mmol) of iridium chloride trihydrate, 3.00 g (9.95 mmol) of the ligand, and 20 ml of ethylene glycol were placed in a 100-ml recovery flask. The mixture was irradiated with microwaves (150 W) for 1 hour. Water was added to the reaction mixture. The mixture was filtered by suction. Dispersion washing was repeated with methanol to give 220 mg of compound (4) as a yellow solid. Analysis by HPLC indicated a purity of 99.5%. The structure was identified by MS and ¹H NMR.
MS analysis: 1093.377
¹H NMR analysis (500 MHz, CDCl₃): 9.03 (1H, d, J/Hz = 1.5), 7.80 (1H, d, J/Hz = 8.0), 7.57 (1H, d, J/Hz = 8.0), 7.52 (1H, d, J/Hz = 6.0), 7.39 (1H, d, J/Hz = 8.0), 7.35 (1H, dt, J/Hz = 8.0, 1.0), 7.26 (1H, dt, J/Hz = 8.0, 1.0), 6.96 (1H, dd, J/Hz = 6.0, 2.0), 6.90 (1H, d, J/Hz = 8.0), 1.41 (9H, s)

### [Synthesis Example 2]

### Synthesis of Compound (13)

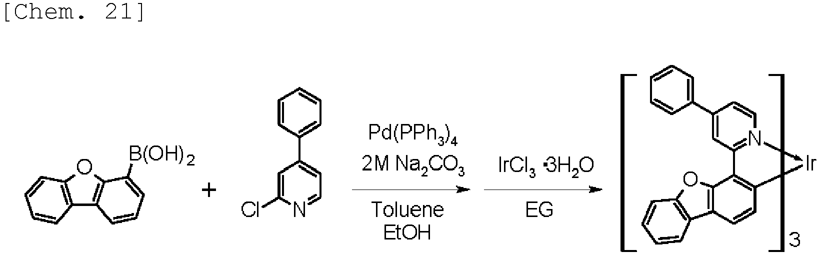

Compound (13) was synthesized in the same manner as in Synthesis Example 1, except that 4-phenyl-2-chloropyridine was used in place of 4-(tert-butyl)-2-chloropyridine. Analysis by HPLC indicated a purity of 99.4%. The structure was identified by MS.
MS analysis: 1153.280

### [Synthesis Example 3]

### Synthesis of Compound (56)

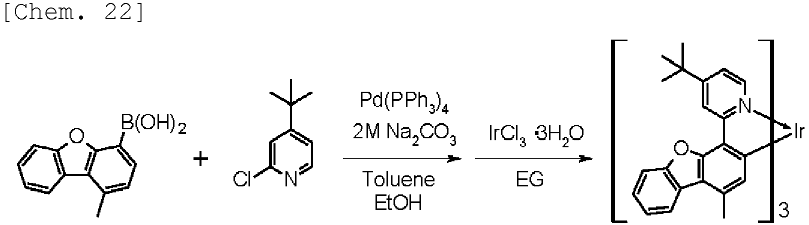

Compound (56) was synthesized in the same manner as in Synthesis Example 1, except that 1-methyldibenzofuran-4-boronic acid was used in place of dibenzofuran-4-boronic acid. Analysis by HPLC indicated a purity of 99.2%. The structure was identified by MS.
MS analysis: 1135.424

### [Synthesis Example 4]

### Synthesis of Compound (58)

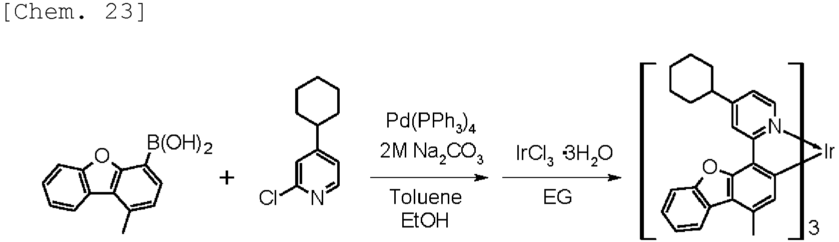

Compound (58) was synthesized in the same manner as in Synthesis Example 1, except that 4-cyclohexyl-2-chloropyridine was used in place of 4-(tert-butyl)-2-chloropyridine. Analysis by HPLC indicated a purity of 99.5%. The structure was identified by MS.
MS analysis: 1213.475

### [Synthesis Example 5]

### Synthesis of Compound (70)

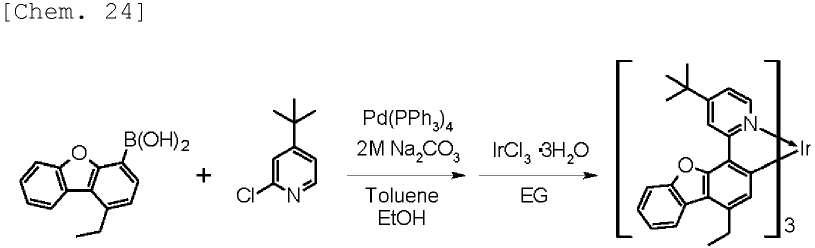

Compound (70) was synthesized in the same manner as in Synthesis Example 1, except that 1-ethyldibenzofuran-4-boronic acid was used in place of dibenzofuran-4-boronic acid. Analysis by HPLC indicated a purity of 99.2%. The structure was identified by MS.
MS analysis: 1177.474

### [Example 1]

Compound (4) prepared in Synthesis Example 1 was purified by sublimation at 360°C in 3 × 10⁻³ Pa. The HPLC purity of the sublimate was 99.9%. Using the sublimate, an organic light-emitting device having a structure of anode/hole injection layer/hole transport layer/electron-blocking layer/light-emitting layer/hole-blocking layer/electron transport layer/cathode, provided in that order, on a substrate was produced as follows.

An ITO film, serving as an anode, having a thickness of 100 nm was formed on a glass substrate by a sputtering method, and the resulting substrate was used as a transparent conductive supporting substrate (ITO substrate). Organic compound layers and electrode layers described below were successively formed over the ITO substrate by vacuum deposition using resistance heating in a vacuum chamber with a pressure of 1 × 10⁻⁵ Pa. At this time, the electrodes were formed so as to have a facing electrode area of 3 mm².
Hole injection layer (10 nm) HT16
Hole transport layer (40 nm) HT1
Light-emitting layer (30 nm) host material: EM32, guest material: compound (4) (4% by weight)
Electron transport layer (30 nm) ET20
Metallic electrode layer 1 (15 nm) LiF
Metallic electrode layer 2 (100 nm) Al

In order not to cause the deterioration of the organic light-emitting device due to moisture adsorption, the resulting structure was covered with a protective glass plate in a dry air atmosphere and sealed with an acrylic resin adhesive.

The current efficiency of the resulting organic light-emitting device was measured using the ITO electrode as an anode and the Al electrode as a cathode. The current efficiency at a current density of 5 mA/cm² was 94.8 cd/A. The current efficiency at a current density of 50 mA/cm² was 79.2 cd/A.

### [Example 2]

Compound (13) prepared in Synthesis Example 2 was purified by sublimation in the same manner as in the case of compound (4) of Example 1 to give a sublimate having an HPLC purity of 99.8%. An organic light-emitting device was produced in the same manner as in Example 1, except that the sublimate of Example 2 was used in place of the sublimate of Example 1.

The current efficiency of the resulting organic light-emitting device was measured using the ITO electrode as an anode and the Al electrode as a cathode. The current efficiency at a current density of 5 mA/cm² was 95.0 cd/A. The current efficiency at a current density of 50 mA/cm² was 80.5 cd/A.

### [Example 3]

Compound (56) prepared in Synthesis Example 3 was purified by sublimation in the same manner as in the case of compound (4) of Example 1 to give a sublimate having an HPLC purity of 99.9%. An organic light-emitting device was produced in the same manner as in Example 1, except that the sublimate of Example 3 was used in place of the sublimate of Example 1.

The current efficiency of the resulting organic light-emitting device was measured using the ITO electrode as an anode and the Al electrode as a cathode. The current efficiency at a current density of 5 mA/cm² was 91.1 cd/A. The current efficiency at a current density of 50 mA/cm² was 77.9 cd/A.

### [Example 4]

Compound (58) prepared in Synthesis Example 4 was purified by sublimation in the same manner as in the case of compound (4) of Example 1 to give a sublimate having an HPLC purity of 99.9%. An organic light-emitting device was produced in the same manner as in Example 1, except that the sublimate of Example 4 was used in place of the sublimate of Example 1.

The current efficiency of the resulting organic light-emitting device was measured using the ITO electrode as an anode and the Al electrode as a cathode. The current efficiency at a current density of 5 mA/cm² was 92.5 cd/A. The current efficiency at a current density of 50 mA/cm² was 75.5 cd/A.

### [Example 5]

Compound (70) prepared in Synthesis Example 5 was purified by sublimation in the same manner as in the case of compound (4) of Example 1 to give a sublimate having an HPLC purity of 99.9%. An organic light-emitting device was produced in the same manner as in Example 1, except that the sublimate of Example 5 was used in place of the sublimate of Example 1.

The current efficiency of the resulting organic light-emitting device was measured using the ITO electrode as an anode and the Al electrode as a cathode. The current efficiency at a current density of 5 mA/cm² was 93.0 cd/A. The current efficiency at a current density of 50 mA/cm² was 78.1 cd/A.

### [Reference Example 1]

Ir(ppy)₃ was purified by sublimation in the same manner as in the case of compound (4) of Example 1 to give a sublimate having an HPLC purity of 99.9%. An organic light-emitting device was produced in the same manner as in Example 1, except that the sublimate of Reference Example 1 was used in place of the sublimate of Example 1.

The current efficiency of the resulting organic light-emitting device was measured using the ITO electrode as an anode and the Al electrode as a cathode. The current efficiency at a current density of 5 mA/cm² was 75.0 cd/A. The current efficiency at a current density of 50 mA/cm² was 62.3 cd/A.

### [Comparative Example 1]

Comparative compound (1) was purified by sublimation in the same manner as in the case of compound (4) of Example 1 to give a sublimate having an HPLC purity of 99.9%. An organic light-emitting device was produced in the same manner as in Example 1, except that the sublimate of Comparative Example 1 was used in place of the sublimate of Example 1.

The current efficiency of the resulting organic light-emitting device was measured using the ITO electrode as an anode and the Al electrode as a cathode. The current efficiency at a current density of 5 mA/cm² was 85.1 cd/A. The current efficiency at a current density of 50 mA/cm² was 65.8 cd/A.

### [Comparative Example 2]

Comparative compound (2) was purified by sublimation in the same manner as in the case of compound (4) of Example 1 to give a sublimate having an HPLC purity of 99.9%. An organic light-emitting device was produced in the same manner as in Example 1, except that the sublimate of Comparative Example 3 was used in place of the sublimate of Example 1.

The current efficiency of the resulting organic light-emitting device was measured using the ITO electrode as an anode and the Al electrode as a cathode. The current efficiency at a current density of 5 mA/cm² was 86.9 cd/A. The current efficiency at a current density of 50 mA/cm² was 65.0 cd/A.

### [Comparative Example 3]

Comparative compound (3) was purified by sublimation in the same manner as in the case of compound (4) of Example 1 to give a sublimate having an HPLC purity of 99.9%. An organic light-emitting device was produced in the same manner as in Example 1, except that the sublimate of Comparative Example 4 was used in place of the sublimate of Example 1.

The current efficiency of the resulting organic light-emitting device was measured using the ITO electrode as an anode and the Al electrode as a cathode. The current efficiency at a current density of 5 mA/cm² was 79.3 cd/A. The current efficiency at a current density of 50 mA/cm² was 62.0 cd/A.

### [Comparative Example 4]

Comparative compound (4) was purified by sublimation in the same manner as in the case of compound (4) of Example 1 to give a sublimate having an HPLC purity of 99.8%. An organic light-emitting device was produced in the same manner as in Example 1, except that the sublimate of Comparative Example 4 was used in place of the sublimate of Example 1.

The current efficiency of the resulting organic light-emitting device was measured using the ITO electrode as an anode and the Al electrode as a cathode. The current efficiency at a current density of 5 mA/cm² was 84.8 cd/A. The current efficiency at a current density of 50 mA/cm² was 66.5 cd/A.

### [Comparative Example 5]

Comparative compound (5) was purified by sublimation in the same manner as in the case of compound (4) of Example 1 to give a sublimate having an HPLC purity of 99.6%. An organic light-emitting device was produced in the same manner as in Example 1, except that the sublimate of Comparative Example 5 was used in place of the sublimate of Example 1.

The current efficiency of the resulting organic light-emitting device was measured using the ITO electrode as an anode and the Al electrode as a cathode. The current efficiency at a current density of 5 mA/cm² was 86.1 cd/A. The current efficiency at a current density of 50 mA/cm² was 67.7 cd/A.

### [Comparative Example 6]

Comparative compound (6) was purified by sublimation in the same manner as in the case of compound (4) of Example 1 to give a sublimate having an HPLC purity of 99.6%. An organic light-emitting device was produced in the same manner as in Example 1, except that the sublimate of Comparative Example 6 was used in place of the sublimate of Example 1.

The current efficiency of the resulting organic light-emitting device was measured using the ITO electrode as an anode and the Al electrode as a cathode. The current efficiency at a current density of 5 mA/cm² was 85.5 cd/A. The current efficiency at a current density of 50 mA/cm² was 64.0 cd/A.

The structural formulae of the comparative compounds are illustrated below.

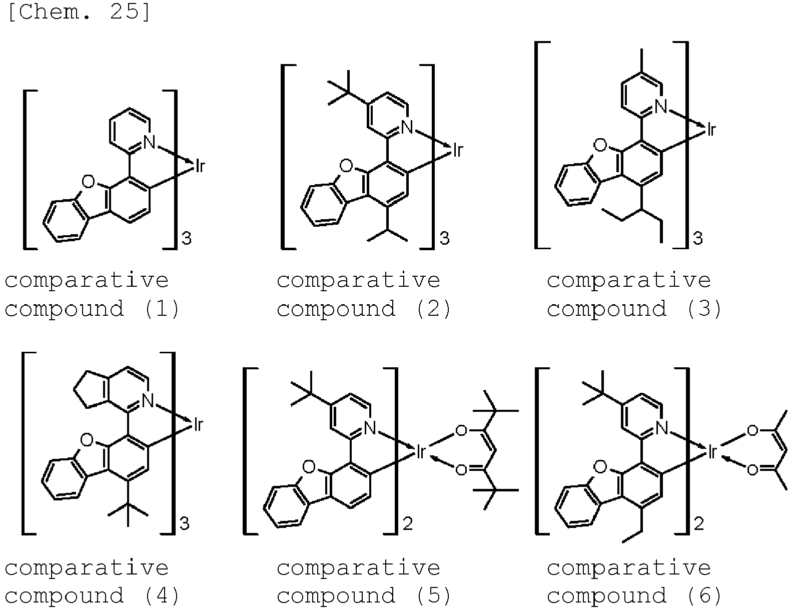

### [Table 1]

**Table 1**

| | GD | CE(cd/A)@5mA/cm² | CE(cd/A)@50mA/cm² | Rate of decrease |
|---|---|---|---|---|
| Example 1 | compound (4) | 94.8 | 79.2 | 16.5% |
| Example 2 | compound (13) | 95.0 | 80.5 | 15.3% |
| Example 3 | compound (56) | 91.1 | 77.9 | 14.5% |
| Example 4 | compound (58) | 92.5 | 75.5 | 18.4% |
| Example 5 | compound (70) | 93.0 | 78.1 | 16.0% |
| Reference Example 1 | Ir(ppy)3 | 75.0 | 62.3 | 16.9% |
| Comparative Example 1 | comparative compound (1) | 85.1 | 65.8 | 22.7% |
| Comparative Example 2 | comparative compound (2) | 86.9 | 65.0 | 25.2% |
| Comparative Example 3 | comparative compound (3) | 79.3 | 62.0 | 21.8% |
| Comparative Example 4 | comparative compound (4) | 84.8 | 66.5 | 21.6% |
| Comparative Example 5 | comparative compound (5) | 86.1 | 67.7 | 21.4% |
| Comparative Example 6 | comparative compound (6) | 85.5 | 64.0 | 25.1% |

### [Example 6]

Using compound (4) described in Example 1, an organic light-emitting device having a structure of anode/hole injection layer/light-emitting layer/electron transport layer/cathode, provided in that order, on a substrate was produced as follows.

An ITO film, serving as an anode, having a thickness of 100 nm was formed on a glass substrate by a sputtering method, and the resulting substrate was used as a transparent conductive supporting substrate (ITO substrate). The ITO substrate was washed with pure water and then with IPA, subjected to UV-ozone treatment, and then subjected to spin coating to form a hole injection layer. The film-forming conditions were as follows.
Coating liquid: solution of poly(3,4-ethylenedioxythiophene)-poly(styrene sulfonate) in water (solution of PEDOT;PSS in water, available from Aldrich, conductivity: 1 × 10⁻⁵ S/cm, concentration of compound: 2.8% by mass)
Spin coating conditions: 3,000 rpm, 60 seconds, in nitrogen atmosphere
Annealing conditions: 200°C, 1 hour, in nitrogen atmosphere
Film thickness: 40 nm

Next, a light-emitting layer was formed by a spin coating method. The composition of the coating solution for forming the light-emitting layer and the film-forming conditions are as follows.
Coating solution: 6.0% by mass of compound (4)
   EM37 94.0% by mass
   Chlorobenzene 9900% by mass
Spin coating conditions: 3,000 rpm, 60 seconds, in nitrogen atmosphere
Annealing conditions: 110°C, 10 minutes, in nitrogen atmosphere
Film thickness: 30 nm

Finally, an electron transport layer and an electrode layer were formed by a vacuum evaporation method using resistance heating in a vacuum chamber of 1 × 10⁻⁵ Pa. At this time, the electrodes were formed so as to have a facing electrode area of 3 mm². The film-forming conditions are as follows.
Electric transport layer: (50 nm) TPBi
Metallic electrode layer 1: (0.5 nm) LiF
Metallic electrode layer 2: (90 nm) Al

Thereafter, in order not to cause the deterioration of the organic light-emitting device due to moisture adsorption, the resulting structure was covered with a protective glass plate in a nitrogen atmosphere and sealed with an acrylic resin adhesive.

The current efficiency of the resulting organic light-emitting device was measured using the ITO electrode as an anode and the Al electrode as a cathode. The current efficiency at a current density of 5 mA/cm² was 55.9 cd/A. The current efficiency at a current density of 50 mA/cm² was 45.4 cd/A.

### [Example 7]

An organic light-emitting device was produced in the same manner as in Example 6, except that compound (97) having an HPLC purity of 99.8% was used in place of compound (4) in Example 6.

The current efficiency of the resulting organic light-emitting device was measured using the ITO electrode as an anode and the Al electrode as a cathode. The current efficiency at a current density of 5 mA/cm² was 53.5 cd/A. The current efficiency at a current density of 50 mA/cm² was 44.2 cd/A.

### [Reference Example 2]

An organic light-emitting device was produced in the same manner as in Example 6, except that Ir(ppy)3 used in Reference Example 1 was used in place of compound (4) in Example 6.

The current efficiency of the resulting organic light-emitting device was measured using the ITO electrode as an anode and the Al electrode as a cathode. The current efficiency at a current density of 5 mA/cm² was 35.2 cd/A. The current efficiency at a current density of 50 mA/cm² was 29.1 cd/A.

### [Comparative Example 7]

An organic light-emitting device was produced in the same manner as in Example 6, except that comparative compound (1) used in Comparative Example 1 was used in place of compound (4) in Example 6.

### [Comparative Example 8]

An organic light-emitting device was produced in the same manner as in Example 6, except that comparative compound (3) used in Comparative Example 3 was used in place of compound (4) in Example 6.

The current efficiency of the resulting organic light-emitting device was measured using the ITO electrode as an anode and the Al electrode as a cathode. The current efficiency at a current density of 5 mA/cm² was 53.0 cd/A. The current efficiency at a current density of 50 mA/cm² was 40.5 cd/A.

### [Comparative Example 9]

An organic light-emitting device was produced in the same manner as in Example 6, except that comparative compound (5) used in Comparative Example 5 was used in place of compound (4) in Example 6.

The current efficiency of the resulting organic light-emitting device was measured using the ITO electrode as an anode and the Al electrode as a cathode. The current efficiency at a current density of 5 mA/cm² was 56.8 cd/A. The current efficiency at a current density of 50 mA/cm² was 42.3 cd/A.

### [Table 2]

**Table 2**

| | GD | CE(cd/A)@5mA/cm² | CE(cd/A)@50mA/cm² | Rate of decrease |
|---|---|---|---|---|
| Example 6 | compound (4) | 55.9 | 45.4 | 18.8% |
| Example 7 | compound (97) | 53.5 | 44.2 | 17.4% |
| Reference Example 1 | Ir(ppy)3 | 35.2 | 29.1 | 17.3% |
| Comparative Example 7 | com parative compound (1) | 52.9 | 40.9 | 22.7% |
| Comparative Example 8 | com parative compound (3) | 53.0 | 40.5 | 23.6% |
| Comparative Example 9 | com parative compound (5) | 56.8 | 42.3 | 25.5% |

As described above, the organometallic complex according to the present invention is an organometallic complex having excellent roll-off characteristics in which the rate of decrease in luminous efficiency at a second current density higher than a first current density is low with respect to the luminous efficiency at the first current density.

The present invention is not limited to the above embodiments and various changes and modifications can be made within the spirit and scope of the present invention. Therefore, to apprise the public of the scope of the present invention, the following claims are made.

This application claims priority based on Japanese Patent Application No. 2021-112295 filed July 6, 2021, which is hereby incorporated by reference herein in its entirety. Reference Signs List

- 1: interlayer insulating layer
- 2: reflective electrode
- 3: insulating layer
- 4: organic compound layer
- 5: transparent electrode
- 6: protective layer
- 7: color filter
- 10: subpixel
- 11: substrate
- 12: insulating layer
- 13: gate electrode
- 14: gate insulating film
- 15: semiconductor layer
- 16: drain electrode
- 17: source electrode
- 18: thin-film transistor
- 19: insulating film
- 20: contact hole
- 21: lower electrode
- 22: organic compound layer
- 23: upper electrode
- 24: first protective layer
- 25: second protective layer
- 26: organic light-emitting device
- 100: display apparatus
- 1000: display apparatus
- 1001: upper cover
- 1002: flexible printed circuit
- 1003: touch panel
- 1004: flexible printed circuit
- 1005: display panel
- 1006: frame
- 1007: circuit board
- 1008: battery
- 1009: lower cover
- 1100: image pickup apparatus
- 1101: viewfinder
- 1102: rear display
- 1103: operation unit
- 1104: housing
- 1200: electronic apparatus
- 1201: display unit
- 1202: operation unit
- 1203: housing
- 1300: display apparatus
- 1301: frame
- 1302: display unit
- 1303: base
- 1310: display apparatus
- 1311: first display portion
- 1312: second display portion
- 1313: housing
- 1314: inflection point
- 1400: lighting apparatus
- 1401: housing
- 1402: light source
- 1403: circuit board
- 1404: optical film
- 1405: light diffusion unit
- 1500: automobile
- 1501: tail lamp
- 1502: window
- 1503: automobile body
- 1600: smart glasses
- 1601: lens
- 1602: image pickup apparatus
- 1603: control unit
- 1610: smart glasses
- 1611: lens
- 1612: control unit

## Claims

1. An organometallic complex represented by the following general formula (1): 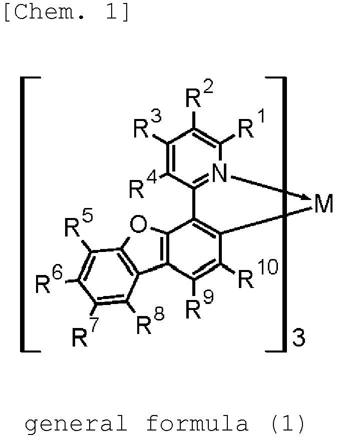 where in general formula (1), M is a metal atom selected from Ir, Pt, Os, Rh, Pd, and Ru, R¹ to R⁸ are each independently selected from a hydrogen atom and a substituent, the substituent is a halogen atom, a cyano group, a nitro group, a trialkylsilyl group, a substituted or unsubstituted alkyl group, a substituted or unsubstituted cycloalkyl group, or a substituted or unsubstituted aryl group having two or less rings, R⁹ and R¹⁰ are each independently selected from a hydrogen atom and an alkyl group having two or less carbon atoms, provided that at least one of R¹ to R⁸ is the substituent, or at least any of R⁹ and R¹⁰ is the alkyl group having two or less carbon atoms.

2. An organometallic complex represented by the following general formula (1): 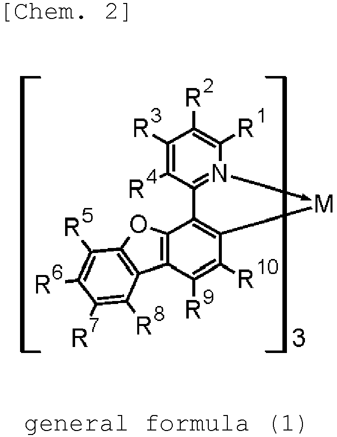 where in general formula (1), M is a metal atom selected from Ir, Pt, Os, Rh, Pd, and Ru, R¹, R², and R⁴ to R⁸ are each independently selected from a hydrogen atom and a substituent, the substituent is a halogen atom, a cyano group, a nitro group, a trialkylsilyl group, a substituted or unsubstituted alkyl group, a substituted or unsubstituted cycloalkyl group, or a substituted or unsubstituted phenyl group, R³ is selected from a hydrogen atom, a halogen atom, a cyano group, a nitro group, a trialkylsilyl group, a substituted or unsubstituted alkyl group, a substituted or unsubstituted cycloalkyl group, and a substituted or unsubstituted aryl group, and R⁹ and R¹⁰ are each independently selected from a hydrogen atom and an alkyl group having two or less carbon atoms, provided that R¹ to R⁸ are not hydrogen atoms, or at least any of R⁹ and R¹⁰ is the alkyl group having two or less carbon atoms.

3. An organometallic complex represented by the following general formula (1): 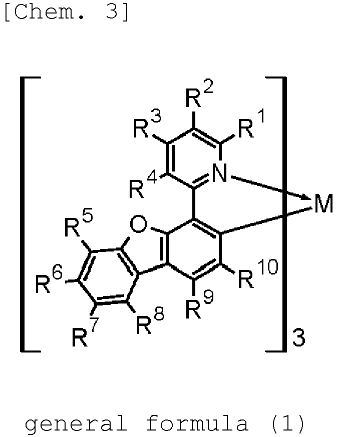 where in general formula (1), M is a metal atom selected from Ir, Pt, Os, Rh, Pd, and Ru, R¹ to R⁸ are each independently selected from a hydrogen atom and a substituent, the substituent is a halogen atom, a cyano group, a nitro group, a trialkylsilyl group, a substituted or unsubstituted alkyl group, a substituted or unsubstituted cycloalkyl group, or a substituted or unsubstituted aryl group, R⁹ and R¹⁰ are each independently selected from a hydrogen atom and an alkyl group having two or less carbon atoms, provided that at least any of R⁹ and R¹⁰ is the alkyl group having two or less carbon atoms.

4. The organometallic complex according to any one of Claims 1 to 3, wherein R¹ to R⁴ are each independently selected from a hydrogen atom and the alkyl group.

5. The organometallic complex according to any one of Claims 1 to 4, wherein R⁵ to R⁸ is a hydrogen atom or the alkyl group.

6. The organometallic complex according to any one of Claims 1 to 5, wherein R⁹ is the alkyl group having two or less carbon atoms.

7. The organometallic complex according to Claim 1 or 2, wherein each of R⁹ and R¹⁰ is a hydrogen atom.

8. The organometallic complex according to any one of Claims 1 to 7, wherein R³ is a tert-butyl group.

9. The organometallic complex according to any one of Claims 1 to 8, wherein the metal atom is Ir.

10. The organometallic complex according to any one of Claims 1 to 9, wherein the organometallic complex is represented by any of the following structural formulae (1) to (5) :

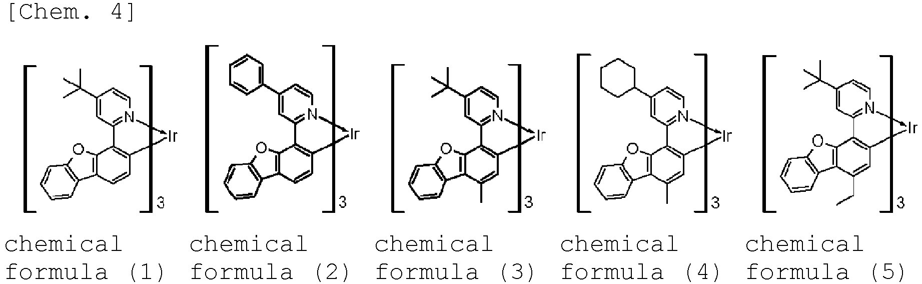

11. An organic light-emitting device, comprising: a first electrode, a second electrode, and an organic compound layer disposed between the first electrode and the second electrode,
wherein the organic compound layer contains the organometallic complex according to any one of Claims 1 to 10.

12. The organic light-emitting device according to Claim 11, wherein the organic compound layer is a light-emitting layer, the light-emitting layer further contains a first organic compound, and
the first organic compound is a compound having a higher lowest excited triplet energy than the organometallic complex.

13. The organic light-emitting device according to Claim 12, wherein the light-emitting layer further contains a second organic compound, and a lowest excited triplet energy of the second organic compound is equal to or higher than the lowest excited triplet energy of the organometallic complex and equal to or lower than the lowest excited triplet energy of the first organic compound.

14. The organic light-emitting device according to Claim 12 or 13, wherein the organic compound layer further includes a first charge transport layer disposed between the first electrode and the light-emitting layer, and a second charge transport layer disposed between the second electrode and the light-emitting layer,
the first electrode is in contact with the first charge transport layer, and the second electrode is in contact with the second charge transport layer.

15. The organic light-emitting device according to Claim 14, wherein the lowest excited triplet energy of the first charge transport layer is higher than the lowest excited triplet energy of the first organic compound, and the lowest excited triplet energy of the second charge transport layer is higher than the lowest excited triplet energy of the first organic compound.

16. A display apparatus, comprising:
multiple pixels, at least one of the multiple pixels including the organic light-emitting device according to any one of Claims 11 to 15, and a transistor coupled to the organic light-emitting device.

17. An image pickup apparatus, comprising an optical unit including multiple lenses, an image pickup device configured to receive light passing through the optical unit, and a display unit configured to display an image captured by the image pickup device,
wherein the display unit includes the organic light-emitting device according to any one of Claims 11 to 15.

18. An electronic apparatus, comprising a display unit including the organic light-emitting device according to any one of Claims 11 to 15, a housing provided with the display unit, and a communication unit being disposed in the housing and communicating with an outside.

19. A lighting apparatus, comprising a light source including the organic light-emitting device according to any one of Claims 11 to 15, and a light diffusion unit or an optical film configured to transmit light emitted from the light source.

20. A moving object, comprising a lighting unit including the organic light-emitting device according to any one of Claims 11 to 15, and a body provided with the lighting unit.
